# EUROPÄISCHE PATENTANMELDUNG

(11) **EP 2 374 535 A1**
(43) Veröffentlichungstag der Anmeldung: **12.10.2011**
(21) Anmeldenummer: 10159108.9
(22) Anmeldetag: 06.04.2010
(51) Int. Cl.: B01J 13/04, A61K 8/11, A61K 9/50, C09B 67/00

(54) **Verfahren und Vorrichtungen zur Vesikelbildung, insbesondere unter Verwendung von Block-Coplymeren**

(71) Anmelder: Bühler AG, 9240 Uzwil (CH)
(72) Erfinder: Bohm, Arturo, 9242, Oberuzwil (CH); Lisner, Jochen, 9532, Rickenbach (CH); Braun, Jörg, 79539, Lörrach (DE); Engel, Helen Alicia, 8624, Grüt (CH); Windhab, Erich Josef, 8261, Hemishofen (CH); Meier, Wolfgang, 4106, Therwil (CH)
(74) Vertreter: Hepp Wenger Ryffel AG

(57) **Zusammenfassung**

Die vorliegende Erfindung betrifft Verfahren zur Herstellung von Vesikeln (3). Dabei wird ein erstes Fluid (1) mit einer amphiphilen Substanz durch eine Pore (21;21';41;51;62) geführt, so dass sich mindestens ein Tropfen (4) aus dem ersten Fluid (1) bildet. Der Tropfen (4) wird anschliessend mittels Vorbeiführens eines zweiten Fluids (2) abgetrennt. Die chemische Zusammensetzung des zweiten Fluids (2) ist von der chemischen Zusammensetzung des ersten Fluids (1) verschieden. Weiterhin betrifft die Erfindung Gesamtzusammensetzungen, die Vesikel (3) enthalten. Speziell betrifft die vorliegende Erfindung Vesikel (3) aus amphiphilen Block-Copolymeren, wobei das Block-Copolymer insbesondere eine Ester- und/oder eine Ether-Funktionalität umfasst. Zudem werden Vorrichtungen zur Herstellung von Vesikeln (3) sowie die Verwendung solcher Vorrichtungen beschrieben.

## Beschreibung

Die vorliegende Erfindung betrifft Vesikel, insbesondere aus Block-Copolymeren gebildete Vesikel. Weiterhin werden Verfahren zur Herstellung von insbesondere unilamellaren Vesikeln beschrieben. Insbesondere betrifft die vorliegende Erfindung Vesikel aus amphiphilen Block-Copolymeren, worin das Block-Copolymer insbesondere eine Ester- und/oder eine Ether-Funktionalität umfasst. Somit werden Vesikel bereitgestellt, die ihrerseits zur Verkapselung bzw. Umhüllung und zum Transport von Wirkstoffen, bspw. ernährungsphysiologisch wichtigen Komponenten oder Arzneimitteln, Kosmetika und Nahrungsmittelbestandteilen, verwendet werden können. Die Entwicklung der vorliegenden Erfindung wurde mitfinanziert durch die KTI (Kommission für Technologie und Innovation).

Vesikel bestehen aus einer geschlossenen Anordnung von einer Doppelschicht von oberflächenaktiven Molekülen und bilden u. a. selbstorganisierende und selbstaggregierende Kügelchen oder Kapseln, wobei in deren Innerem insbesondere hydrophile Wirkstoffmoleküle eingelagert sein können und in deren Randbereich insbesondere hydrophobe Moleküle vorhanden sein können. Vesikel sind meist sphärisch geformte Lipidbläschen, die aus einer einzigen oder mehreren Lipid-Doppelschichten gebildet werden und im Inneren eine wässrige Phase einschließen. Sie werden aufgrund ihrer unterschiedlichen Größe und Anzahl der Membranschichten in grober Näherung in die Gruppen multilamellarer Vesikel (MLV; mehr als 0,5 µm), kleiner unilamellarer Vesikel (SUV; 0,02 bis 0,05 µm) und großer unilamellarer Vesikel (LUV; 0,06 bis 0,5 µm) eingeteilt. Da deren Bildung zuerst bei Phospholipiden beobachtet wurde, setzte sich, insbesondere für Vesikel auf Basis von Phospholipiden, auch die Bezeichnung Liposome durch.

Folgende Vorteile lassen sich durch Verkapselung von Wirkstoffen gegenüber den herkömmlichen Zubereitungen erzielen:
a) Schutz des Wirkstoffes vor vorzeitigem Abbau (in vivo und in vitro),
b) Anlegen eines Wirkstoffdepots mit gezielter Wirkstofffreisetzung,
c) Verminderung der Toxizität von Wirkstoffen,
d) Erhöhung der Wirksamkeit bestimmter Wirkstoffe,
e) spezifische Gewebeverteilung der wirksamen Bestandteile (natürliches "Zielorgan" RES), und
f) Überführung lipophiler Wirkstoffe in eine wasserlösliche Form (ursprünglich wasserunlösliche Stoffe werden nach Verpacken in Vesikel besser wasserlöslich).

Für eine Übersicht dieser und weiterer Vorteile des Einsatzes wirkstoffbeladener Liposomen siehe u. a. Mihalko, P. J., Schreier, H., Abra, R.M., Liposomes as Drug Carriers (G. Gregoriadis ed.), Wiley and Sons, New York 1988, insbesondere S. 679-694.
Weitere vorteilhafte Eigenschaften von Nanokapseln bzw. in Vesikeln verkapselten Wirkstoffen liegen darin, dass sie oftmals nicht sichtbar sind, da die Abmessungen kleiner sind als der sichtbare Wellenlängenbereich, und dass eine Sedimentation und eine erhöhte Resorption vermieden werden, wie beispielsweise bei Liposomen in Hautcremes, bei denen davon auszugehen ist, dass der penetrationsfördernde Effekt von topisch applizierten Liposomen auf eine Interaktion zwischen Vesikelsystem und Haut zurückzuführen ist. Auch ist bei Nanokapseln das Verhältnis von Oberfläche zu Volumen um 10er-Potenzen grösser als bei anderen Kapseln.

Jede einzelne Vesikelart kann durch unterschiedliche Verfahren hergestellt werden. Naturgemäß sind nur einige wenige Verfahren geeignet, wirkstoffbeladene Vesikel herzustellen, die beispielsweise in Arzneimitteln zum Einsatz kommen können. 1975 wurde von J. M. Gebicki und M. Hicks erstmals über die Bildung einer Doppelschicht-Membranstruktur in Vesikeln berichtet, die durch die Verwendung synthetischer amphiphiler Materialien gebildet wurden. Weitere Verfahren zur Herstellung von Vesikeln bestehen im Dispergieren in wässriger Phase von konzentrierten Lösungen geeigneter amphiphiler Verbindungen in organischen Lösungsmitteln (Ether, Chloroform, Ethanol durch Einspritzen). Auf diese Weise können auch große Vesikel mit Wänden aus einer oder mehreren Lamellen gewonnen werden.

Bei der Behandlung wässriger Suspensionen amphiphiler organischer Verbindungen mit Ultraschall werden ebenfalls sphärische bimolekulare oder monomolekulare Schichten gebildet. Die Bildung von Vesikeln aus zwei amphiphilen Verbindungen wird beispielsweise in EP 1878766, FR 2677897 und WO 2006/049497 beschrieben, die als kennzeichnende Funktionalitäten beispielsweise eine Aminogruppe oder Thiophengruppe aufweisen.

Patentanmeldungen jüngeren Datums zielen auf die Verwendung von Drei-Block-Copolymeren ab. So wird in WO 2006/049497 und WO 2007/008300 die Verwendung von ABC-Copolymeren beschrieben, die sich zu einem Vesikel selbstaggregierend anlagern.

Die Vesikel dienen überwiegend dazu, Wirkstoffe einzuschließen und stellen somit Träger für diese Wirkstoffe dar. Die Wirkstoffe können niedermolekulare Stoffe sein, beispielsweise Arzneistoffe, oder auch hochmolekulare Stoffe, beispielsweise Proteine. Aufgrund dieser zunehmenden Bedeutung, die Vesikel zum Einschluss und zum Transport von Wirkstoffen durch lebendes Gewebe, z. B. durch die Haut, in der Medizin und in der Kosmetik erlangt haben, werden ständig neue vesikelbildende Substanzen und Verfahren zur Herstellung von geeigneten Vesikeln und Vesikel-Dispersionen gesucht.

So wurde von Rahman et al. in J. Lab. Clin. Med. 83, 640 (1974) der Einschluss von Ethylendiamintetraacetat in Liposomen beschrieben, die zur Behandlung von Schwermetall-Vergiftungen verwendet werden können und deutlich bessere Behandlungsergebnisse als die Injektion von nicht eingeschlossenem EDTA zeigen. Insulin (FEBS letters 62, 60 (1976)), Radionuklide, Interferon, Hormone und andere die Körperfunktionen unterstützende Biomoleküle (EP-PS 0 009 842) lassen sich ebenfalls in Vesikel einschließen und dem Körper zuführen. Auch aus synthetischen, phospholipidanalogen Verbindungen werden, wie beispielsweise in der GB 20 20 663, Vesikel zur Verkapselung von pharmazeutischen Wirkstoffen hergestellt. Von Nachteil ist dabei die schwere Zugänglichkeit solcher vesikelbildender Verbindungen. Es ist ferner bekannt, aus symmetrischen, amphiphilen Molekülen mit hydrophilen Endgruppen an einem lipophilen Kohlenwasserstoffgerüst Vesikel mit monomolekularen Schichten dadurch herzustellen, dass man der Lipidmembran durch Einführung von Abstandshaltern, die im Sinne eines molekularen Keils in die Lipidmembranen eingebaut werden, eine Kurvatur aufzwingt (vgl. Y. Okahata und T. Kunnitake, J. Am. Chem. Soc. 101, 5231 (1979)). Auch die hierfür bisher bekannten amphiphilen Verbindungen sind schwer zugänglich.
Die US 3 957 971 offenbart ferner die äußere, lokale Anwendung von Feuchtigkeitsspendern für die Haut, in denen Liposomen aus Sojabohnen-Lecithin, Dicethylphosphat und Sterinen die aktiven Wirkstoffe enthalten. In der US 4 217 344 wird die Herstellung von Vesikeln beschrieben, die hygroskopische Substanzen verkapseln und über ihr osmotisches Verhalten das Wasserrückhaltevermögen der Haut erhöhen.

Eine besondere Bedeutung haben Ausgangsstoffe, die Vesikel mit höherer Stabilität gegenüber störenden Bestandteilen und gegenüber mechanischer und thermischer Belastung bilden können. Weiterhin sollten derartige Ausgangsstoffe die hohen Anforderungen in Pharmazie, Kosmetik und Lebensmittelindustrie gegenüber beispielsweise Reinheit und Verträglichkeit, aber auch gegenüber dem zu transportierenden Wirkstoff erfüllen.

Bei bekannten Herstellungsverfahren wird ein eine amphiphile Substanz enthaltendes erstes Fluid durch eine Membran gepresst. Nach dem Durchströmen der Membran bilden sich Tropfen, die von einem zweiten Fluid abgetrennt werden, so dass sich Vesikel bilden. Bei den bekannten Verfahren stimmen das erste Fluid und das zweite Fluid mit Ausnahme der im ersten Fluid enthaltenen amphiphilen Substanz in ihrer chemischen Zusammensetzung überein. Dies ist beispielsweise in "Extrusion Technique to Generate Liposomes of Defined Size" von B. Mui el al., Methods in Enzymology, Vol. 367, S. 3 ff. beschrieben.

Hier und im Folgenden beinhaltet der Begriff "Zusammensetzung" stets sowohl die Angabe der Inhaltsstoffe als auch deren Konzentrationen. Demgemäß unterscheiden sich also die chemischen Zusammensetzungen des ersten Fluids und des zweiten Fluids, wenn nur das erste Fluid einen gewissen Inhaltsstoff enthält, das zweite Fluid diesen Inhaltsstoff aber nicht enthält. Die chemischen Zusammensetzungen des ersten Fluids und des zweiten Fluids unterscheiden sich aber auch bereits dann, wenn die Inhaltsstoffe in beiden Fluiden identisch sind, aber die Konzentrationen mindestens eines dieser Inhaltsstoffe in den beiden Fluiden voneinander verschieden sind.

Insbesondere sind bei diesen bekannten Verfahren die Wirkstoffkonzentrationen im Vesikels und im zweiten Fluid identisch. Das Vesikel umfasst dabei sowohl eine die amphiphile Schicht enthaltene äussere Schicht und einen Innenbereich, der von dieser Schicht eingeschlossen wird. Bei diesem Verfahren wird eine Gesamtzusammensetzung erhalten, die einerseits die aus dem ersten Fluid gebildeten Vesikel enthält und andererseits das zweite Fluid, welches als Trägerfluid für die Vesikel dient.

Der Anteil des Wirkstoffs im Trägerfluid liegt in unverkapselter Form vor und ist einem vorzeitigen und unkontrollierten Abbau ausgesetzt. Lediglich der in den Vesikeln eingeschlossene Anteil des Wirkstoffs kann kontrolliert freigesetzt werden. Der Anteil des Wirkstoffs im Trägerfluid kann also überflüssig oder (beispielsweise bei medizinischen Anwendungen) sogar schädlich sein. Zwar ist es möglich, die Gesamtzusammensetzung und damit auch die Konzentration des Wirkstoffs im Trägerfluid zu verdünnen. Hierdurch wird jedoch ein zusätzlicher Verdünnungsschritt erforderlich, was das Verfahren aufwändig macht. Dieser Nachteil wurde beispielsweise in "Spherulites: A new vesicular system with promising applications. An example: Enzyme microencapsulation" von A. Bernheim-Grosswasser et al., Journal of Chemical Physics, Vol. 112, Number 7, S. 3424 ff. beschrieben, aber nicht überwunden.

Weiterhin ist aus US 5,762,957 ein Verfahren zur Herstellung einer Gesamtzusammensetzung bekannt, bei dem eine Anreicherung eines Wirkstoffes im Innenbereich eines Vesikels mit Hilfe eines pH-Gradienten zwischen dem Innenbereich und Trägerfluid erzielt wird. Auch hier ist also ein zusätzlicher Schritt nach der eigentlichen Herstellung der Vesikel erforderlich. Ausserdem müssen die Membranen der Vesikel für den Wirkstoff durchlässig sein, was die Auswahl der amphiphilen Substanz und des Wirkstoffes erheblich einschränkt. Darüber hinaus muss auch ein ausreichender pH-Gradient vorhanden sein, was die Möglichkeiten ebenfalls begrenzt.

Die vorliegende Erfindung stellt sich die Aufgabe, Vesikel mit einem schnellen, einfachen, kostengünstigen und kontinuierlichen Verfahren herzustellen, bei dem die aus dem Stand der Technik bekannten Probleme überwunden werden.

Gemäß bevorzugten Ausführungsformen sollen Vesikel einer definierten Größenverteilung und insbesondere mit möglichst identischen Abmessungen in einem einfachen Verfahren erzeugt werden. Weiterhin sollen bevorzugt Vesikel aus einem preiswert verfügbaren Material hergestellt werden, wobei die entstehenden Produkte eine verbesserte Einschlusskapazität aufweisen sollen. Mit der Einschlusskapazität ist dabei die Menge eines im Vesikel enthaltenen Wirkstoffs gemeint. Wegen ihres denkbaren Einsatzes auch auf anderen Gebieten, z. B. bei der Verkapselung von Enzymen, Bleichaktivatoren oder antimikrobiellen Wirkstoffen im Bereich flüssiger Wasch-, Reinigungs- und Desinfektionsmittel oder für das Verkapseln flüchtiger Duftstoffe in Parfums, ist in einigen Ausführungsformen auch eine geringere Empfindlichkeit gegenüber tensidhaltigen Medien und organischen Lösungsmitteln wünschenswert.

Es wurde nun gefunden, dass die vorgenannten Bedingungen in besonders hohem Maße erfüllt werden, wenn ein Verfahren eingesetzt wird, das die folgenden Schritte umfasst:
(a) Bereitstellen eines ersten Fluids, welches zumindest eine amphiphile Substanz enthält;
(b) Durchführen des ersten Fluids durch mindestens eine Pore von einer ersten Seite eines porösen Materials zu einer zweiten Seite des porösen Materials;
(c) Bildung eines Tropfens aus dem ersten Fluid an der zweiten Seite des porösen Materials;
(d) Bildung mindestens eines Vesikels durch Abtrennung, insbesondere Abscherung des Tropfens mittels Vorbeiführens eines zweiten Fluids an der zweiten Seite des porösen Materials, wobei die chemische Zusammensetzung des zweiten Fluids von der chemischen Zusammensetzung des ersten Fluids verschieden ist.

Das poröse Material kann dabei beispielsweise eine Membran sein, also im Wesentlichen flächig ausgebildet sein. Unter einem Tropfen des ersten Fluids wird hier und im Folgenden stets eine Ausstülpung des ersten Fluids verstanden, welche gegenüber dem zweiten Fluid durch die im ersten Fluid enthaltene amphiphile Substanz abgegrenzt ist. Das zweite Fluid ermöglicht sowohl ein Ablösen als auch einen Abtransport des gebildeten Vesikels, so dass ein neues Vesikel an der zweiten Seite des porösen Materials gebildet werden kann.

Dieses erfindungsgemäße Verfahren ist sowohl schnell, einfach, kostengünstig und kann überdies kontinuierlich durchgeführt werden. Ferner können hohe Ausbeuten und langzeitstabile Vesikel erhalten werden.

Durch das erfindungsgemäße Verfahren werden
Gesamtzusammensetzungen aus einem vom zweiten Fluid gebildeten Trägerfluid und darin enthaltenen Vesikeln aus dem ersten Fluid erhalten. Da sich die chemische Zusammensetzung des zweiten Fluids von der des ersten Fluids unterscheidet, können die Eigenschaften der Gesamtzusammensetzung gezielt und unabhängig voneinander eingestellt werden. Die chemische Zusammensetzung des ersten Fluids und damit auch die Zusammensetzung des erzeugten Vesikels ist damit praktisch unabhängig von der Umgebung, in welche das erste Fluid extrudiert wird, also von der chemischen Zusammensetzung des zweiten Fluids.

Weiterhin bevorzugt umfasst das erste Fluid mindestens einen insbesondere zu verkapselnden Wirkstoff. Dieser kann beispielsweise im ersten Fluid suspendiert oder gelöst sein. Beim erfindungsgemäßen Verfahren kann somit die gesonderte Verkapselung oder Umhüllung des mindestens einen Wirkstoffs entfallen. Hierdurch können diesbezügliche Techniken, wie z. B. "remote loading" über einen pH-Gradienten,
Hochdruckhomogenisierung oder "ethanol-injection method" (Injektion einer Ethanol-Lipid-Lösung in die wässrige Wirkstofflösung) vermieden werden.
Im Sinne der obigen Definition des Begriffes "Zusammensetzung" können sich die chemischen Zusammensetzungen des ersten Fluids und des zweiten Fluids dadurch unterscheiden, das nur das erste Fluid einen gewissen Inhaltsstoff enthält, das zweite diesen Inhaltsstoff aber nicht enthält. Umgekehrt kann auch nur das zweite Fluid einen gewissen Inhaltsstoff enthalten, das erste Fluid jedoch nicht.

Es liegt jedoch auch im Rahmen der Erfindung, dass die Inhaltsstoffe im ersten Fluid und im zweiten Fluid identisch sind, aber die Konzentration mindestens eines Inhaltsstoffes im ersten Fluid von der Konzentration dieses Inhaltsstoffes im zweiten Fluid abweicht. Insbesondere kann es sich bei diesem Inhaltsstoff um einen Wirkstoff handeln.

Bevorzugt enthält das erste Fluid mindestens einen Inhaltsstoff in einer ersten Konzentration, und das zweite Fluid enthält den Inhaltsstoff in einer zweiten Konzentration, die von der ersten Konzentration verschieden ist. Insbesondere kann die zweite Konzentration kleiner sein als die erste Konzentration. Besonders bevorzugt ist das zweite Fluid im Wesentlichen frei von dem Inhaltsstoff; in diesem Falle ist die Konzentration des Inhaltsstoff im zweiten Fluid null. Insbesondere kann es sich bei dem Inhaltsstoff um einen Wirkstoff handeln.

Auf diese Weise ist es ohne einen weiteren Verdünnungsschritt möglich, Gesamtzusammensetzungen aus einem Trägerfluid und darin enthaltenen Vesikeln herzustellen, bei denen ein Grossteil des Wirkstoffes in den Vesikeln enthalten ist, insbesondere darin verkapselt ist, und daher kontrolliert abgegeben werden kann. Bevorzugt beträgt die zweite Konzentration des Wirkstoffs weniger als 80 %, weiter bevorzugt weniger als 50 %, besonders bevorzugt weniger als 30 % der ersten Konzentration des Wirkstoffs. Beträgt also beispielsweise die erste Konzentration des Wirkstoffs 30 % und beträgt die zweite Konzentration des Wirkstoffs weniger als 80 % der ersten Konzentration, so beträgt die zweite Konzentration des Wirkstoffs weniger als 24 %.

Das erste Fluid enthält die Ausgangsstoffe zur Vesikelbildung. Bevorzugt umfasst das erste Fluid ein Lösungsmittel, insbesondere Wasser. Das Fluid kann in einem ersten Strömungsraum an die erste Seite des porösen Materials herangeführt werden.
Das erste Fluid gemäß dem erfindungsgemäßen Verfahren enthält eine oder mehrere amphiphile Substanzen. Das Wort "amphiphil" beschreibt die chemische Eigenschaft einer Substanz, sowohl hydrophil als auch lipophil zu sein. Das heißt, sie ist sowohl in polaren Lösungsmitteln als auch in unpolaren Lösungsmitteln löslich. Dies beruht darauf, dass die Moleküle sowohl hydrophile als auch hydrophobe Bereiche aufweisen.

Das bekannteste polare Lösungsmittel ist Wasser, worauf die Bezeichnung "hydrophil" zurückgeht. Eine Art der Hydrophobie kann sein, dass sich die Substanz gut in Fetten oder Ölen löst; die Substanz wird dann lipophil genannt.

Wichtige amphiphile Substanzen sind
- Tenside, die als Seifen benutzt werden. Sie haben gerade aufgrund der Amphiphilie die Eigenschaft, reinigend zu wirken;
- Emulgatoren in Lebensmitteln, zum Beispiel Lecithin;
- Lipide mit polaren Kopfgruppen, insbesondere Phospholipide, ein Hauptbestandteil der Zellmembranen;
- Block-Copolymere.

Eine wichtige Eigenschaft amphiphiler Stoffe ist ihre Tendenz zur Selbstorganisation, z. B. die Bildung von Mizellen und Vesikeln, bei höheren Konzentrationen auch von geordneten Phasen.

Alle Tenside sind aus einem unpolaren und einem polaren Teil (Funktionelle Gruppen) aufgebaut (Polarität). Als unpolarer Teil dient immer eine Alkylgruppe. Der polare Teil kann verschieden aufgebaut sein und ist in der folgenden Tabelle zusammengefasst.

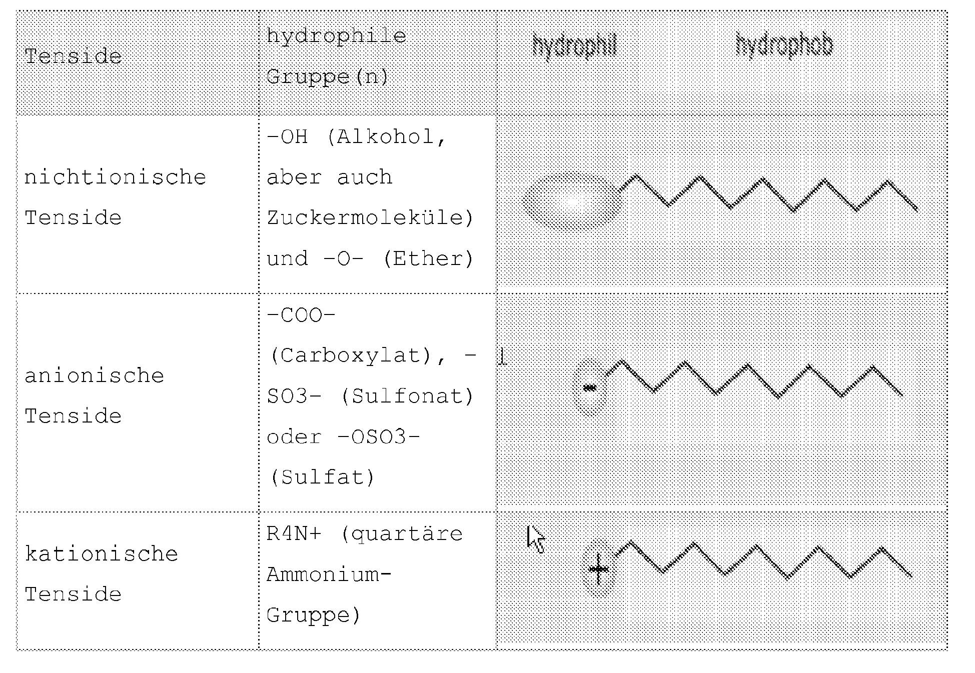

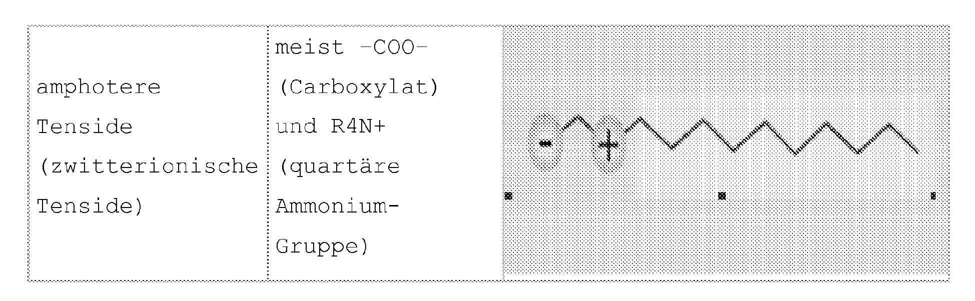

Phospholipide sind phosphorhaltige, amphiphile Lipide. Sie sind im Organismus als Membranlipide am Aufbau der Doppellipidschicht einer Biomembran beteiligt. Sie setzen sich aus einem hydrophilen Kopf und zwei hydrophoben Kohlenwasserstoffschwänzen zusammen. Sie sind somit amphiphil.

Phospholipide bilden in Wasser eine Doppellipidschicht (Liposom). Die häufigsten in einer Zellmembran auftretenden Phospholipide sind Phosphatidylcholine (auch Lecithine, kurz PC), Phosphatidylethanolamine (PE), Phosphatidylserine (PS) und Sphingomyeline.

Phospholipide gliedern sich aufgrund ihres chemischen Aufbaus in die folgenden zwei Gruppen:
- Phosphoglyceride mit Glycerin als Grundgerüst (auch Glycerophospholipide oder Phosphatide genannt);
- Sphingolipide, die vom Sphingosin abgeleitet sind (u.a. auch Sphingomyeline).
Es existieren weiterhin so genannte Plasmalogene. Diese unterscheiden sich von Phosphoglyceriden nur dadurch, dass sie am C₁-Atom des Glycerins statt einer Fettsäure einen über eine Etherbrücke verknüpften, ungesättigten Alkohol (z. B. -O-CH=CH-(CH₂)ₙ-CH₃) tragen.

Einige wenige Beispiele von Lipiden sind die folgenden:
- DAG Di-acylglycerol
- SM Sphingomyelin
- DAPC Di-arachioylphosphatidylcholin
- DCPC Di-decanoylphosphatidylcholin
- DLPC Di-laureoylphosphatidylcholin
- DMPC Di-myristoylphosphatidylcholin
- DOPC Di-oleoylphosphatidylcholin
- DPPC Di-Palmitoylphosphatidylcholin
- DSPC Di-stearoylphosphatidylcholin
- POPC Palmitoyloleoylphosphatidylcholin
- SOPC Stearoyloleoylphosphatidylcholin
- DMPE Di-myristoylphosphatidylethanolamin
- DPPE Di-palmitoylphosphatiddylethanolamin
- DSPE Di-stearoylphosphatidylethanolamin
- POPE Palmitoyloleoylphosphatidylethanolamine
- DMPG Di-myristoylphosphatidylglycerol

Das erste Fluid kann mindestens eine Pufferlösung und/oder mindestens ein Tensid und/oder mindestens ein Salz und/oder mindestens eine Säure und/oder mindestens eine Lauge umfassen. Hierdurch kann die Stabilität des Vesikels eingestellt werden und insbesondere der Zeitraum eingestellt werden, innerhalb dessen ein im Vesikel enthaltener Wirkstoff freigesetzt wird.

Bevorzugt liegt das erste Fluid zumindest vor dem Durchführen durch die Pore in einer geordneten Phase vor. Unter geordneten Phasen werden dabei die Phasen mit der Nomenklatur nach Luzzati verstanden, so wie sie im Abschnitt II.6 in D. Marsh, Handbook of Lipid Bilayers dargestellt sind:
- L = eindimensional lamellar ("one-dimensional, lamellar") ;
- P = zweidimensional schief oder zentriert ("two-dimensional oblique or centered (rippled)");
- H = zweidimensional hexagonal ("two-dimensional hexagonal") ;
- H_{I} = normal, Öl in Wasser ("normal, oil-in-water");
- H_{II} = invertiert, Wasser in Öl ("inverted, water-inoil") ;
- R = rhomboedrisch ("rhombohedral");
- Q = kubisch ("cubic");
- C = dreidimensional, kristallin ("three-dimensional, crystalline");
- L_{c} = kristallin lamellar.

Die Phasen können beispielsweise mittels Röntgenbeugung, Freeze-Fracture Electron Microscopy oder ³¹P-NMR identifiziert werden.

Bevorzugt liegt das erste Fluid in einer lamellaren Phase vor, besonders bevorzugt in einer eindimensional lamellaren ("one-dimensional lamallar") Phase. Falls das erste Fluid in einer geordneten Phase vorliegt, insbesondere in einer eindimensional lamellaren Phase, erfolgt die Herstellung der Vesikel unmittelbar aus der lamellaren Phase des ersten Fluids. Mit anderen Worten entsteht das Vesikel mit dem gewünschten Durchmesser und dem/den darin enthaltenen Wirkstoff(en) unmittelbar und nicht wie bei anderen Verfahren des Stands der Technik über einen oder mehrere Zwischenschritte, die oftmals zu großen multilamellaren Vesikeln führen, die sich ihrerseits erst in einem nachfolgenden Schritt zu den gewünschten kleineren unilamellaren Vesikeln umlagern. Bei einer derartigen aus dem Stand der Technik bekannten Umlagerung können die multilamellaren Vesikel den Wirkstoff an die Umgebung abgeben oder mit dem Lösungsmittel austauschen, so dass unilamellare Vesikel uneinheitlicher Beladung in Bezug auf den Wirkstoff und die Menge desselben erhalten werden.
Bevorzugt wird die Temperatur und/oder der pH-Wert und/oder die Ionenzahl und/oder die Ionenstärke des ersten Fluids und/oder die Konzentrationen der Inhaltsstoffe im ersten Fluid derart eingestellt, dass die Bildung von Vesikeln im Schritt (d) begünstigt wird. Insbesondere können die Temperatur und/oder der pH-Wert und oder die Ionenzahl und/oder die Ionenstärke des ersten Fluids derart eingestellt werden, dass das erste Fluid zumindest vor der Durchführung durch die Pore in einer lamellaren Phase vorliegt.

Optional kann das erste Fluid eine zeitlich variable Strömungsgeschwindigkeit aufweisen. Hierdurch werden Trägheitskräfte wirksam, welche die Bildung von Vesikeln beeinflussen und insbesondere erleichtern können. Darüber hinaus können sie die weiter unten beschriebene Bildung von Tropfen aus dem ersten Fluid und deren Abtrennung zur Bildung von Vesikeln beeinflussen.
Das poröse Material kann beispielsweise ein Sintermetall, eine Membran oder ein poröses Glas sein. Die Herstellung von geeigneten Sintermetallen, Membranen und porösen Gläsern mit erwünschter Porengröße bzw. Verteilung der Porengröße ist dem Fachmann an sich gut bekannt. Geeignete poröse Sintermetalle sind beispielsweise von der Firma Portec Ltd., 8355 Aadorf, Schweiz unter dem Markennamen METAPOR® erhältlich. Ebenfalls geeignete poröse Sintermetalle aus Bronze sind von der Firma Meyer Sintermetall AG, 2557 Studen, Schweiz erhältlich. Weitere geeignete nanoporöse Metallmembranen wurden vom Institut für Werkstoffe der Technischen Universität Braunschweig, Deutschland entwickelt. Geeignete nanoporöse Glasmembranen werden etwa von der CPI - ChemiePark Institut GmbH in 06749 Bitterfeld, Deutschland angeboten. Als Membranen können beispielsweise auch herkömmliche Sterilfilter einer geeigneten Porengröße eingesetzt werden. Weitere geeignete poröse Materialien umfassen beispielsweise poröse Kunststoffe.

Gemäß noch einer weiteren Ausführungsform weist mindestens eine Pore, bevorzugt mindestens 30 % der Poren, weiter bevorzugt mindestens 50 % der Poren, besonders bevorzugt mindestens 75 % der Poren einen Durchmesser von 50 nm bis 10000 nm, bevorzugt 250 nm bis 1000 nm, besonders bevorzugt 450 nm bis 550 nm auf. Derartige Durchmesser sind insbesondere für die Herstellung von unilamellaren Vesikeln geeignet, insbesondere beim erfindungsgemässen Vorliegen des ersten Fluids in einer geordneten Phase.
Besonders bevorzugt beträgt für mindestens eine Pore, bevorzugt mindestens 30 % der Poren, weiter bevorzugt mindestens 50 % der Poren, besonders bevorzugt mindestens 75 % der Poren das Verhältnis aus Länge der Pore und der Durchmesser der Pore mindestens 5, bevorzugt mindestens 10, besonders bevorzugt mindestens 20.

Weiterhin bevorzugt beträgt für mindestens eine Pore, bevorzugt mindestens 10 % der Poren, weiter bevorzugt mindestens 50 % der Poren, besonders bevorzugt mindestens 80 % der Poren das Verhältnis aus Länge der Pore und der Durchmesser der Pore höchstens 500, bevorzugt höchstens 200, besonders bevorzugt höchstens 50.

Weiterhin können die Poren mit mindestens einer hydrophoben oder mindestens einer hydrophilen Substanz beschichtet sein, so dass die mechanische Beanspruchung der Vesikel bei ihrer Bildung eingestellt werden kann.

In bevorzugten Ausführungsformen beträgt die Dicke des porösen Materials, insbesondere der Membran, höchstens 10 µm, bevorzugt höchstens 5 µm, besonders bevorzugt höchstens 2 µm.

Es wurde überraschend gefunden, dass durch Durchführen des die zur Vesikelbildung benötigten Ausgangstoffe enthaltenden Fluids durch Poren bestimmter definierter Abmessungen ebenso Vesikel definierter Abmessungen und geringer Abweichung von der mittleren Größe erhalten werden.

Bevorzugt wird ein poröses Material mit Poren verwendet, deren Durchmesser zwischen 6 % und 14 %, mehr bevorzugt zwischen 7 % und 13 %, zwischen 8 % und 12 %, zwischen 9 % und 11 % und am meisten bevorzugt bei 10 % über den erwünschten Durchmessern der Vesikel liegen. Hierdurch können Vesikel einer relativ schmalen Größenverteilung erzeugt werden.

Alternativ kann auch ein poröses Materialien mit verschiedenen Porendurchmessern verwendet werden, um Vesikel mit anderen, breiteren Größenverteilung herzustellen. Dies kann beispielsweise bei der gleichzeitigen Verkapselung von Wirkstoffen verschiedener Größe von Interesse sein.

Das Durchführen des ersten Fluid durch die mindestens eine Pore im Schritt (b) kann mittels dem Fachmann geläufiger Techniken erfolgen. Hierzu zählen beispielsweise die Zentrifugation und die Verwendung von Druckgradienten. In einigen Ausführungsformen wird Schritt (b) mittels Zentrifugation durchgeführt. Das erste Fluid wird hierbei aufgrund von Zentrifugalkräften durch die Pore oder Poren des porösen Materials gedrückt.

Das zweite Fluid kann in einem zweiten Strömungsraum an der zweiten Seite des porösen Materials vorbeigeführt werden. Bei geeigneter Dimensionierung des zweiten Strömungsraumes kann eine kontrollierte laminare Strömung erreicht werden. Hierdurch können Verwirbelungen vermieden werden, die ihrerseits zu Vesikeln verschiedener Größe führen können.

Optional kann das zweite Fluid eine zeitlich variable Strömungsgeschwindigkeit aufweisen. Die Strömungsgeschwindigkeit wird hierbei als vektorielle Größe verstanden; auch eine Strömungsgeschwindigkeit, bei der sich lediglich die Richtung, nicht aber auch der Betrag zeitlich ändert, wird also als zeitlich variabel angesehen. Dabei lässt sich das Ablösen der Tropfen durch eine Scherströmung oder eine Dehnströmung oder einer Überlagerung der Strömungsformen im zweiten Fluid steuern.

Das zweite Fluid kann beispielsweise Wasser enthalten oder daraus bestehen.

In einigen Ausführungsformen kann das zweite Fluid mindestens eine Pufferlösung und/oder mindestens ein Tensid und/oder mindestens ein Salz und/oder mindestens eine Säure und/oder mindestens eine Lauge umfassen. Mit Hilfe dieser Inhaltsstoffe können die Bedingungen eingestellt werden, welche für die amphiphile Substanz erforderlich sind. Beim Abtrennen eines Tropfens entsteht eine Abtrennfläche, welche zunächst nicht von der amphiphilen Substanz überdeckt ist. Durch geeignete Wahl und Konzentration der Inhaltsstoffe im zweiten Fluid kann bewirkt werden, dass diese Abtrennfläche schnell von einer Doppelschicht der amphiphilen Substanz überdeckt wird, so dass sich ein stabiles Vesikel bildet.

Bevorzugt wird die Temperatur und/oder der pH-Wert und oder die Ionenzahl und/oder die Ionenstärke des zweiten Fluids derart eingestellt, dass die Bildung von Vesikeln im Schritt (c) begünstigt wird.

Die Bildung des Vesikels in Schritt (d) kann beispielsweise auch mittels Aufbringens einer Scherströmung oder einer Dehnströmung oder deren Kombination davon im zweiten Fluid erreicht oder begünstigt werden.

Zusätzlich kann die Bildung des Vesikels in Schritt (d) auch mittels einer mechanischen Vorrichtung, insbesondere mittels einer Scherplatte, durchgeführt werden. Insbesondere kann eine erste Seite der Scherplatte entlang der zweiten Seite des porösen Materials vorbeigeführt werden, wodurch beispielsweise Tropfen mittels der Scherplatte entlang der zweiten Seite des porösen Materials abgetrennt werden. Bevorzugt wird das zweite Fluid entlang einer zweiten Seite der Scherplatte vorbeigeführt.

Ebenfalls alternativ oder zusätzlich kann zur Bildung des Vesikels in Schritt (d), insbesondere zur Abtrennung eines Tropfens, das erste Fluid und/oder das zweite Fluid und/oder das poröse Material in mechanische Schwingungen versetzt und/oder mit mechanischen Wellen beaufschlagt werden. Von Vorteil ist, wenn die Frequenz der Schwingungen oder Wellen in der Nähe einer Eigenfrequenz der zu bildenden Vesikel liegt. Das zweite Fluid kann also ein resonierendes Strömungsfeld aufweisen.

In einigen Ausführungsformen werden das erste Fluid und/oder das zweite Fluid und/oder das poröse Material mittels Ultraschalls in mechanische Schwingungen versetzt und/oder mit mechanischen Wellen beaufschlagt. Der Ultraschall kann mit einem an sich bekannten Ultraschall-Resonator erzeugt werden.

Bevorzugt umfasst die amphiphile Substanz des ersten Fluids mindestens ein amphiphiles Block-Copolymer, das aus zwei oder mehreren verschiedenen Blöcken bestehen kann. Mindestens einer dieser Blöcke muss hydrophob und mindestens einer muss hydrophil sein. Beispielsweise sind die Kombinationen AB, ABA oder BAB möglich, wobei A ein hydrophober und B ein hydrophiler Block ist. Um in hydrophilen Umgebungen (Lösungsmitteln) Vesikel bilden zu können, müssen die hydrophoben Eigenschaften die hydrophilen überwiegen. In hydrophoben Medien ist dies umgekehrt.

Beispiele für hydrophile Blöcke sind Polyalkylenoxid, Polymethyl-oxazolin, Polyacrylsäure, Polysacharide und Polyhydroxyethylmethylacrylat. Bei dem Polyalkylenoxid kann es sich beispielsweise um Polyethylenoxid handeln.

Beispiele für hydrophobe Blöcke sind Polyalkylcaprolacton, Polyisobutylen, Polyalkylacrylat, Polybutadien, Polyethylen, Polyisopren, Polydimethylsulfoxid, Polystyrol. Bei dem Polyalkylcaprolacton kann es sich beispielsweise um Polymethylcaprolacton handeln.

Besonders bevorzugt umfasst das Block-Copolymer mindestens eine Ester-Funktionalität und/oder mindestens eine Ether-Funktionalität, die für den amphiphilen Charakter der Block-Copolymere verantwortlich sind.

Gemäß einer weiteren Ausführungsform ist das amphiphile Block-Copolymer ausgewählt aus der Gruppe bestehend aus Poly(alkylenoxid)ₙ-Poly(alkylcaprolacton)ₘ und Poly(isobutylen)ₙ-Poly(ethylenoxid)ₘ. Dabei ist "Poly(alkylenoxid)ₙ" bevorzugt "Poly(ethylenoxid)ₙ". Weiterhin ist "Poly(alkylcaprolacton)ₘ" bevorzugt "Poly(methylcaprolacton)ₙ". Für Poly(alkylenoxid)ₙ-Poly(alkylcaprolacton)ₘ, insbesondere für Poly(ethylenoxid)ₙ-Poly(methylcaprolacton)ₘ, liegt n bevorzugt im Bereich von 10 bis 50, und m liegt bevorzugt im Bereich von 20 bis 80. Für Poly(isobutylen)ₙ-Poly(ethylenoxid)ₘ liegt n bevorzugt im Bereich von 30 bis 100, und m liegt bevorzugt im Bereich von 10 bis 50.

Besonders bevorzugt weist das Poly(ethylenoxid)ₙ-Poly(methylcaprolacton)ₘ eine Struktur nach folgender Formel I auf: 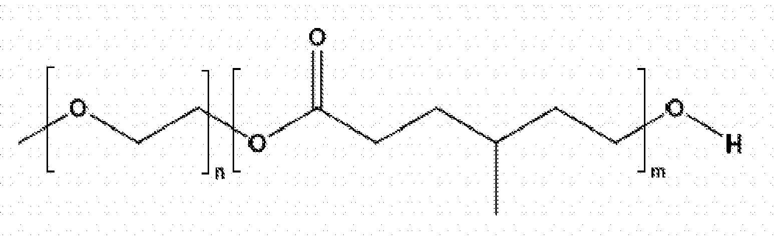 , wobei n bevorzugt im Bereich von 10 bis 50 liegt und m bevorzugt im Bereich von 20 bis 80 liegt.

Ebenfalls besonders bevorzugt weist das Poly(isobutylen)ₙ-Poly(ethylenoxid)ₘ eine Struktur nach folgender Formel II auf:

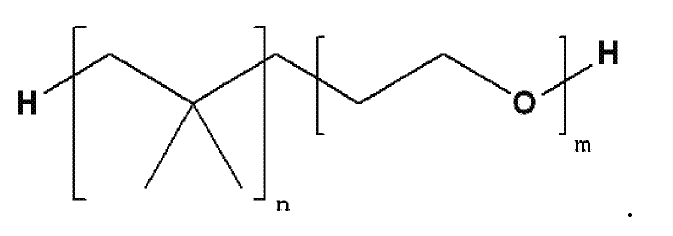

Es wurde gefunden, dass insbesondere Poly(ethylenoxid)₁₀₋₅₀-Poly(methylcaprolacton)₂₀₋₈₀ und Poly(isobutylen)₃₀₋₁₀₀-Poly(ethylenoxid)₁₀₋₅₀ aufgrund der einstellbaren Kettenlängen für die Vesikelbildung besonders geeignet sind, da bei diesen Ausgangsstoffen sowohl die variable Länge der hydrophoben Reste, die Verzweigung der hydrophoben Reste (die die Beweglichkeit des Moleküls in der lamellaren Phase bestimmt) und die Größe der hydrophilen Köpfe die Bildung von unilamellaren Vesikeln definierter Größe mit nur geringen Abweichungen aus der lamellaren Phase ermöglicht.

Ein weiterer Aspekt der Erfindung betrifft eine
Gesamtzusammensetzung, die eine Trägerfluid und mindestens ein Vesikel enthält. Das Vesikel kann mit einem wie oben beschriebenen Verfahren hergestellt worden sein. In diesem Falle kann das zweite Fluid das Trägerfluid bilden.

Zumindest einige der Vesikel in der Gesamtzusammensetzung, bevorzugt im Wesentlichen alle Vesikel in der
Gesamtzusammensetzung können eine oder mehrere der oben beschriebenen Eigenschaften aufweisen. Insbesondere können die Vesikel mindestens eines der oben beschriebenen Block-Copolymere umfassen.
Erfindungsgemäss ist die chemische Zusammensetzung des Vesikels von der chemischen Zusammensetzung des Trägerfluids verschieden.

Bevorzugt enthält das Vesikel mindestens einen Inhaltsstoff in einer ersten Konzentration, während das Trägerfluid den Inhaltsstoff in einer zweiten Konzentration enthält, die von der ersten Konzentration verschieden ist. Bei dem Inhaltsstoff kann es sich insbesondere um einen Wirkstoff handeln. Der Inhaltsstoff, insbesondere der Wirkstoff, kann in der die amphiphile Substanz enthaltenden äusseren Schicht des Vesikels und/oder in dem von dieser Schicht eingeschlossenen Innenbereich des Vesikels vorhanden sein.

Insbesondere kann die zweite Konzentration kleiner sein als die erste Konzentration. Bei einer solchen Gesamtzusammensetzung ist ein Grossteil des Inhaltsstoffes, insbesondere des Wirkstoffes, im Vesikel enthalten und kann daher kontrolliert abgegeben werden.

Bevorzugt beträgt die zweite Konzentration des Inhaltsstoffs weniger als 80 %, weiter bevorzugt weniger als 50 %, besonders bevorzugt weniger als 30 % der ersten Konzentration des Inhaltsstoffs. Beträgt also beispielsweise die erste Konzentration des Wirkstoffs 30 % und beträgt die zweite Konzentration des Wirkstoffs weniger als 80 % der ersten Konzentration, so beträgt die zweite Konzentration des Wirkstoffs weniger als 24 %.

In einigen Ausführungsformen können die pH-Werte im Innenbereich des Vesikels und im Trägerfluid im Wesentlichen gleich sein. Hierunter wird verstanden, dass sich die pH-Werte um höchstens 0,15, bevorzugt höchstens 0,1, besonders bevorzugt höchstens 0,05 voneinander unterscheiden.

Ein weiterer Aspekt der Erfindung betrifft eine Vorrichtung zur Herstellung von Vesikeln. Die Vorrichtung umfasst
- mindestens ein poröses Material, insbesondere eine Membran, und/oder eine Aufnahme für ein poröses Material, insbesondere eine Membran, wobei das Material eine erste Seite und eine zweite Seite sowie mindestens eine Pore aufweist, welche sich von der ersten Seite zur zweiten Seite erstreckt;
- einen ersten Strömungsraum zum Heranführen eines ersten Fluids an die erste Seite des porösen Materials;
- einen zweiten Strömungsraum zum Vorbeiführen eines zweiten Fluids an der zweiten Seite des porösen
Mit einer derartigen Vorrichtung können Vesikel hergestellt werden, insbesondere in dem erfindungsgemässen Verfahren. Mittels eines im zweiten Strömungsraum an der zweiten Seite des porösen Materials vorbei geführten zweiten Fluids können also Tropfen eines ersten Fluids abgetrennt werden, welches aus dem ersten Strömungsraum durch das poröse Material in den zweiten Strömungsraum geführt wurde. Überraschend ist dabei, dass sich bei Verwendung dieser Vorrichtung die Schicht der amphiphilen Substanz bei der Abtrennung der Tropfen mittels des zweiten Fluids um den abzutrennenden Tropfen herum schliesst, so dass beispielsweise keine ungeordneten Strukturen entstehen, sondern Vesikel mit einer geschlossenen Schicht der amphiphilen Substanz.

Erfindungsgemäss ist mindestens eine Dimension des zweiten Strömungsraumes einstellbar, und zwar zumindest mittels einer Bewegung mindestens einer den zweiten Strömungsraum begrenzenden Fläche. Diese Bewegung soll dabei relativ zur zweiten Seite des porösen Materials erfolgen. Dabei ist nicht ausgeschlossen, dass eine Einstellbarkeit der Dimension des zweiten Strömungsraumes auch zusätzlich durch Bewegung des porösen Materials erfolgen kann.

Bevorzugt ist die Dimension des zweiten Strömungsraumes allein mittels einer Bewegung mindestens einer den zweiten Strömungsraum begrenzenden Fläche einstellbar, ohne dass das poröse Material bewegt werden muss. Dies vereinfacht die Zuführung des ersten Fluids erheblich.

Der komplette Einbau und/oder Ausbau und/oder Umbau von Schikanen in den oder aus dem zweiten Strömungsraum wird dabei nicht als Bewegung mindestens einer den zweiten Strömungsraum begrenzenden Fläche verstanden; eine Vorrichtung, bei der die Dimensionen des zweiten Strömungsraumes lediglich durch einen solchen Einbau und/oder Ausbau und/oder Umbau erreicht werden kann, ist von dieser Formulierung daher nicht erfasst.

Aufgrund der Einstellbarkeit mindestens einer Dimension des zweiten Strömungsraumes mittels Bewegung einer den zweiten Strömungsraum begrenzenden Fläche kann können die Strömungseigenschaften des zweiten Fluids auf besonders einfache und schnelle Weise beeinflusst werden. So können etwa die Anteile einer Dehnströmung und/oder einer Scherströmung einfach und schnell eingestellt werden, ohne dass die Vorrichtung aufwändig umgebaut oder sogar komplett ausgetauscht werden muss.

Besonders bevorzugt ist mindestens eine Dimension des zweiten Strömungsraumes im Bereich der zweiten Seite des porösen Materials und im Wesentlichen senkrecht zur zweiten Seite einstellbar, und zwar zumindest mittels einer Bewegung mindestens einer den zweiten Strömungsraum begrenzenden Fläche. Hierdurch können besonders gut die Strömungseigenschaften des zweiten Fluids in dem Bereich eingestellt werden, in dem sich Tropfen aus dem ersten Fluid bilden.

Beispielsweise kann die Breite eines Spaltes des zweiten Strömungsraumes im Bereich der zweiten Seite des porösen Materials einstellbar sein.

Das poröse Material und insbesondere die darin enthaltene Pore kann eine, mehrere oder alle der oben im Zusammenhang mit dem Verfahren diskutierten Eigenschaften aufweisen. Die Vorrichtung kann insbesondere zur Herstellung von Vesikeln mit dem erfindungsgemäßen Verfahren verwendet werden.

Bevorzugt weist die Vorrichtung weitere Mittel zum Abtrennen von an der zweiten Seite des porösen Materials gebildeten Tropfen des ersten Fluids (1) zur Bildung von Vesikeln auf. In einigen Ausführungsformen sind auch die weiteren Mittel zum Abtrennen als Mittel zur Herstellung einer Scherströmung, einer Dehnströmung oder einer beliebigen Kombination davon im zweiten Fluid ausgebildet.

In einigen Ausführungsformen enthält die Vorrichtung einen Hohlkörper, insbesondere einen Hohlzylinder, und eine innerhalb des Hohlkörpers angeordnete drehbare Welle. Der Hohlkörper umfasst
- eine Mantelfläche, welche den zweiten Strömungsraum umschließt und an welcher das poröse Material angeordnet ist
- eine zweiten Einlassöffnung für das zweite Fluid, welche mit dem zweiten Strömungsraum in Verbindung steht;
- eine zweite Auslassöffnung für das zweite Fluid, welche mit dem zweiten Strömungsraum in Verbindung steht.

Dabei ist die Welle mit insbesondere variabler
Umfangsgeschwindigkeit relativ zum Hohlkörper drehbar. Weiterhin ist die Welle gegenüber dem Hohlkörper, insbesondere gegenüber einer Längsachse des Hohlzylinders, exzentrisch verschiebbar. Hierdurch bildet die Welle Mittel zur Herstellung einer Scherströmung, einer Dehnströmung oder einer beliebiger Kombination davon im zweiten Fluid. Durch Wahl der Umfangsgeschwindigkeit und/oder der exzentrischen Verschiebung kann das Verhältnis von Dehnströmung und Scherströmung eingestellt werden. Eine exzentrische Verschiebung der Welle gegenüber dem Hohlkörper führt also zur Einstellung einer Dimension des zweiten Strömungsraumes. Die Oberfläche der Welle bildet dabei die bewegbare, den zweiten Strömungsraum begrenzende Fläche im Sinne der Erfindung.
Bevorzugt ist die erste Einlassöffnung an der Stelle des engsten Spaltes zwischen Hohlkörper und Welle angeordnet. Durch diese erste Einlassöffnung kann das erste Fluid in den zweiten Strömungsraum einströmen. Bevorzugt ist die zweite Einlassöffnung an einer der Strömungsrichtung vorgelagerten Stelle der Mantelfläche angeordnet. Die zweite Auslassöffnung ist bevorzugt an einer der Strömungsrichtung nachgelagerten Stelle der Mantelfläche angeordnet. Durch diese zweite Auslassöffnung kann das zweite Fluid mit darin suspendierten Vesikeln aus dem zweiten Strömungsraum herausgeführt werden.

In anderen Ausführungsformen sind die weiteren Mittel zum Abtrennen der Tropfen als Mittel zum Beaufschlagen des ersten Fluids und/oder des zweiten Fluids und/oder des porösen Materials mit mechanischen Schwingungen und/oder Wellen ausgebildet. Insbesondere kann es sich bei diesen Mittel um mindestens einen Rohrresonator und/oder mindestens einen Piezofilm und/oder mindestens einen Unwuchterzeuger und/oder mindestens einen Elektromagnet handeln.

Die Vorrichtung kann einen Kanal mit mindestens einer Wandfläche aufweisen, wobei der Kanal den ersten Strömungsraum und den zweiten Strömungsraum enthält. Das poröse Material kann als Membran ausgebildet sein und den ersten Strömungsraum vom zweiten Strömungsraum trennen. Insbesondere kann die Membran im Wesentlichen parallel zur Wandfläche angeordnet sein. Die erste Einlassöffnung für das erste Fluid kann an der Wandfläche angeordnet sein, wobei die erste Einlassöffnung in Verbindung mit dem ersten Strömungsraum ist. Die Mittel zur Herstellung von Schwingungen und/oder Wellen können ausgebildet sein als
- mindestens ein Rohrresonator im ersten und/oder zweiten Strömungsraum und/oder
- mindestens ein Piezofilm an mindestens einer Wand des Kanals und/oder
- mindestens ein Piezofilm an der Membran und/oder
- mindestens ein Unwuchterzeuger und/oder mindestens ein Elektromagnet, insbesondere an mindestens einer Wand des Kanals und/oder an der Membran.

Bevorzugt wird der Rohrresonator mit einer Frequenz im Bereich von 2 Hz bis 100 Hz, bevorzugt von 10 Hz bis 20 Hz angeregt.

In weiteren Ausführungsformen können die weiteren Mittel zum Abtrennen als Mittel zum Abscheren der Tropfen entlang der zweiten Seite des porösen Materials ausgebildet sind, insbesondere als mindestens eine Scherplatte.

In weiteren Ausführungsformen umfasst die Vorrichtung
- einen ersten Block, insbesondere einen oberen Block,
- einen zweiten Block, insbesondere einen unteren Block,
- einen Spalt zwischen dem ersten Block und dem zweiten Block, welcher den zweiten Strömungsraum bildet;
- eine Welle zum Drehen des ersten Blocks bezüglich des zweiten Blocks;
- eine im zweiten Block angeordnete und an einer ersten Stelle in den Spalt einmündende erste Einlassöffnung für das erste Fluid, wobei das poröse Material, insbesondere die Membran, an der ersten Stelle angeordnet ist;
- eine im zweiten Block insbesondere zentral in axialer Richtung angeordnete und an einer zweiten, von der ersten verschiedenen Stelle in den Spalt einmündende zweite Einlassöffnung für das zweite Fluid.;
- eine Stelleinrichtung zum Einstellen der Breite des Spaltes.

Die Breite des Spaltes bildet dabei die einstellbare Dimension des zweiten Strömungsraumes, und die den Spalt begrenzende Oberfläche des ersten Blocks und/oder die den Spalt begrenzende Oberfläche des zweiten Blocks bildet die bewegbare, den zweiten Strömungsraum begrenzende Fläche. Die Breite des Spaltes kann etwa durch die axiale Kraft am Rotor und/oder durch den Volumenstrom des zweiten Fluids eingestellt werden.

Vorzugsweise hat die erste Einlassöffnung die Form eines Ringkanals.

Die Drehung des ersten Blocks relativ zum zweiten Block erzeugt im Bereich des Spaltes eine Scherströmung, welche der Strömung in einem Axiallager entspricht. Die Scherrate, also die räumliche Änderung der Strömungsgeschwindigkeit in einer Richtung senkrecht zur Strömungsgeschwindigkeit, resultiert aus der Drehzahl des ersten Blocks relativ zum zweiten Block und aus der Breite des Spaltes.

In noch weiteren Ausführungsformen der Erfindung umfasst die Vorrichtung
- einen insbesondere konischen Hohlkörper mit einer Deckfläche und einer Mantelfläche;
- eine zu einem Abschnitt der inneren Mantelfläche des Hohlkörpers im Wesentlichen gleichmäßig beabstandete und insbesondere konische Membran, welche das poröse Material bildet;
- einen insbesondere konischen Innenkörper, der zumindest teilweise von der Membran umschlossen ist und insbesondere koaxial zum Hohlkörper angeordnet ist;
- eine insbesondere in der Mantelfläche angeordnete erste Einlassöffnung für das erste Fluid;
- eine insbesondere in der Deckfläche angeordnete zweite Einlassöffnung für das zweite Fluid; und
- eine insbesondere eine Grundfläche des Hohlkörpers durchdringende Welle zum Drehen des Innenkörpers bezüglich des Hohlkörpers und zum Verändern des Abstandes zwischen der Membran und dem Innenkörper durch Verschieben des Innenkörpers entlang der Längsachse des Der Abstand zwischen dem konischen Innenkörper und der Membran bildet dabei im Sinne der Erfindung die einstellbare Dimension des zweiten Strömungsraumes, während die konische Oberfläche des konischen Innenkörpers die bewegbare, den zweiten Strömungsraum begrenzende Fläche darstellt.

Durch Ausbildung eines kleinen Öffnungswinkels des insbesondere konischen Innenkörpers und/oder der Membran kann die axiale Kraft auf die Konusse minimiert werden. Die Scherrate resultiert aus dem Abstand zwischen dem konischen Innenkörper und der Membran und der Drehzahl des Rotors.

Besonders bevorzugt weist der konische Innenkörper einen ersten Öffnungswinkel auf, und die Membran weist einen zweiten Öffnungswinkel auf, welcher kleiner ist als der erste Öffnungswinkel. Hierdurch verengt sich ein Spalt zwischen dem konischen Innenkörper und der Membran.

Die Erfindung betrifft weiterhin die Verwendung einer Vorrichtung zum Herstellung von Vesikeln und/oder zur Herstellung einer wie oben beschriebenen Gesamtzusammensetzung. Die Vorrichtung kann in einem wie oben beschriebenen Verfahren verwendet werden. Bei der Verwendung wird erfindungsgemäss eine Vorrichtung eingesetzt, die das folgende aufweist:
- mindestens ein poröses Material, insbesondere eine Membran, und/oder eine Aufnahme für ein poröses Material, insbesondere für eine Membran, wobei das poröse Material eine erste Seite und eine zweite Seite sowie mindestens eine Pore aufweist, welche sich von der ersten Seite (16;22;33) zur zweiten Seite (17;23;34) erstreckt;
- einen ersten Strömungsraum (12;30;93) zum Heranführen eines ersten Fluids (1) an die erste Seite (16;22;33) des porösen Materials (14;31;40;55;72;78;94;105);
- einen zweiten Strömungsraum (15;32;63;71;90;108) zum Vorbeiführen eines zweiten Fluids (2) an der zweiten Seite (17;23;34) des porösen Materials (14;31;40;55;72;78;94;105).

Überdies kann die Vorrichtung auch eines, mehrere oder alle der oben beschriebenen Merkmale aufweisen.

Ein weiterer Aspekt der Erfindung betrifft die Verwendung von Vesikeln, welche mit einem erfindungsgemäßen Verfahren und/oder mit einer erfindungsgemäßen Vorrichtung erzeugt wurden, als oder in einem Medikament, einem Kosmetikum, einem Nahrungsmittel, einer Farbe, einem Kraftstoff oder einem Antikorrosionsmittel. Das in Vesikeln enthaltenen Antikorrosionsmittel kann zum Beispiel bei Verwitterung und/oder Einwirkung von UV-Licht freigesetzt werden und dann seine gewünschte Wirkung entfalten.

Insbesondere sind die folgenden Verwendungen denkbar:
- Vorbereitung, Herstellung und Lagerung von Diagnostika, Kontrastmitteln und Radiotracern für die Krebsdiagnostik;
- als Nanoreaktoren;
- Verwendung bei der Vorbereitung, Erstellung und Herstellung von Mitteln für gentechnologische Methoden und Verfahren, analytische Methoden und Verfahren, immunologische Methoden und Verfahren für In-vivo-Untersuchungen und für In-vitro-Untersuchungen;
- Verwendung zur Herstellung von Produkte für Ernährungs-, pharmazeutische, medizinische, diagnostische, kosmetische, chemische, umweltorientierte, landwirtschaftliche und/oder technische Zwecke;
- Verwendung bei der Vorbereitung, Herstellung und Lagerung von Implantierungsmaterialien und Prothesen.

Bevorzugt sind die Verwendung der erfindungsgemäßen Vesikel und/oder die Vesikel, die gemäß dem erfindungsgemäßen Verfahren erhalten werden, zur Umhüllung/Verkapselung von Wirkstoffen, Wirkstoff-Prodrug, Wirkstoffe-Pro-Prodrug oder Hilfsstoffen für Humanarzneistoffe, Tierarzneistoffe, Impfstoffe, Enzyme, Coenzyme, Isoenzyme, Vitamine, Hormone, Proteine, Peptide, Kohlenhydrate, Naturstoffe, Riechstoffe, strukturverwandte Riechstoffe wie Pheromone und Aromastoffe in der Medizin, in der Kosmetika, als Zahnpflegemittel, Haut- und Haarpflegemittel, Hauttalg und Reinigungsmittel, in Lebensmittel oder Lebensmittelzusätze, Futtermittel, Konservierungsmittel und andere Zusätze, für landwirtschaftliche Zwecke eingesetzte Produkte wie Düngemittel, Biozide wie Pestizide, Herbizide oder Fungizide, Mizellenbildner (Detergentien), Desinfektionsmittel, Metallprodukte, Nicht-Metallprodukte, chemische Produkte, und Umweltprodukten.

Bevorzugte pharmazeutische Anwendungen schließen eine Behandlung/Linderung von Kreislauferkrankungen ein mit Wirkstoffen wie beispielsweise Isoflavon, Vitamin E, Vitamin C und Folsäure. Eine weitere Anwendung besteht in der Behandlung und/oder Vorbeugung von Osteoporose mit einer Kombination von Vitamin D3, Zink, Magnesium und/oder Calcium. Eine noch weitere Anwendung liegt in der Behandlung und/oder Vorbeugung von Verdauungsstörungen mit Fructooligosaccharid und/oder partiell hydrolisiertem Guargummi. Es sollte klar sein, dass dies nur beispielhafte pharmazeutische Anwendungen sind, bei denen einer oder mehrere Wirkstoffe in verkapselter Form vorliegen können.

Die Herstellung von pharmazeutischen Formulierungen, die die Vesikel mit dem Wirkstoff enthalten, erfolgt nach an sich bekannten Verfahren zur Herstellung pharmazeutischer Formulierungen, die für eine orale, paranterale (i.v., s.c., i.m.), rektale, nasale und inhalatorische Applikation geeignet sind. Im Allgemeinen schließen geeignete Arzneiformen ebenso rektale und vaginale Arzneiformen, perkutane, subkutane und transdermale Arzneiformen, Arzneiformen zur Anwendung am Auge, Arzneiformen zur Anwendung in der Nase und dem Ohr, perorale Arzneiformen mit rascher Wirkstofffreisetzung, perorale Arzneiformen mit langsamer Wirkstofffreisetzung, perorale Arzneiformen mit kontrollierter Wirkstofffreisetzung, dentale Applikation oder topische Applikationsformen ein.

Verkapseln oder Umhüllen im Lebensmittelbereich findet zum Schützen der Komponenten und damit zur Erhöhung der Stabilität, der Erhaltung der Verfügbarkeit (z.B. Immobilisierung von flüchtigen Komponenten) und der Maskierung des Geschmacks der Komponenten statt (Gibbs B. F. et al., International Journal of Food Sciences and Nutrition; 50 (1999) 213-224). Durch gesteuerte Freisetzung der Komponenten kann weiterhin deren Resorption verbessert werden (Gouin S.; Trends in Food Science and Technology; 15 (2004) 330-347).

In einigen Ausführungsformen finden die erfindungsgemäßen Vesikel, Verfahren und Vorrichtungen bei der Verkapselung von Wirkstoffen in Flüssigkeiten oder Getränken Anwendung. Eine weitere Verwendung besteht in der Verkapselung von Komponenten von Naturstoffen.

Allgemein können ernährungsphysiologisch relevante Komponenten verkapselt werden, wie etwa Vitamine, bei denen eine gezielte, kontrollierte Freigabe von Interesse ist. Solche schützenwerten Komponenten können empfindlich gegenüber einer Vielzahl von Einflüssen, wie beispielsweise Licht, Sauerstoff, Wasser, pH-Wert sein. Ferner kann es sich um flüchtige Komponenten oder solche Komponenten handeln, die unangenehm schmecken und/oder einen Fehlgeschmack aufweisen, und die somit zu maskieren sind. Alternativ kann es sich um Komponenten handeln, die nur in geringen Mengen benötigt werden, wie beispielsweise Vitamine, Mineralien oder Phytochemikalien.

Vorzugsweise werden Geschmacksstoffe, Redoxagenzien, Enzyme, künstliche Süßstoffe, Gärungsstoffe, Antioxidantien, Farbstoffe, Wirkstoffe mit unangenehmem Geruch oder Geschmack, ätherische Öle, Aminosäuren, Vitamine und Mineralien verkapselt.

Insbesondere können Wirkstoffe wie Phytin verkapselt werden, um eine Freigabe erst im Dickdarm zu ermöglichen. Weiterhin verkapselt werden können Folsäure und Isoflavon als Cholesterol senkende Komponenten, Vitamin D3 und Zink für die Fortifikation des Immunsystems und Stärkung der Knochen, und Insulin für die orale Verabreichung. Andere Anwendungen bestehen in der Verkapselung von Lycopen und seinen Derivaten zur Anwendung in Anti-Ageing-Produkten.

Eine Depot-Anwendung mit langsamer Freisetzung der Wirkstoffe ist möglich. Die Wirkstoffdosierung kann bei konstanter Vesikeldosis variiert werden. Die Bioverfügbarkeit sollte deshalb unabhängig von der Wirkstoffdosierung sein. Eine langsame bzw. kontrollierte Freisetzung des Wirkstoffs ist durch die Wahl der Vesikelgröße möglich.

Es wurde gefunden, dass die erfindungsgemäßen Vesikel oder Vesikel, die nach dem erfindungsgemäßen Verfahren hergestellt wurden, einen hohen Wirkstoffgehalt mit einer Einschlussrate von mindestens 50 %, bevorzugt mindestens 60 %, weiter bevorzugt mindestens 70 %, noch mehr bevorzugt mindestens 80 %, weiter bevorzugt mindestens 90 % oder noch weiter bevorzugt sogar mindestens 95 % aufweisen. Derartige Vesikel können die Halbwertszeit eines Pharmazeutikums im Blut um mehr als das Zehnfache erhöhen.
Die Vesikel können in Mischung mit natürlichen und/oder synthetischen Polymeren aller Art wie Stärke, Stärkehydrolyse-und andere Stärkeabbauprodukte, Polyethylenglycol und anderen löslichen, schwerlöslichen und/oder unlöslichen Polymeren verwendet werden. In einer weiteren Ausführungsvariante der Erfindung enthält die Vesikelpräparation in oder an der Membran der Vesikel ein Polymer, vorzugsweise Polyethylenglycol mit einem mittleren Molekulargewicht (MG) von 2000 bis 10000, das den Wirkstoff an der Vesikeloberfläche vor Interaktionen mit Körperbestandteilen schützt. Zu den Körperbestandteilen zählen insbesondere Blut bei einer Verwendung als Pharmazeutikum, Säure bei Verwendung als Kosmetikum und Nahrungsmittel (Säureschutzmantel der Haut bzw. Magensäure).

In die Vesikelstruktur können im Allgemeinen sowohl wasserlösliche als auch wasserunlösliche Wirkstoffe eingeschlossen werden. Als Transportvehikel von lipophilen und hydrophilen Wirkstoffen werden die Vesikel in vielen Bereichen von Therapie und Diagnostik eingesetzt (Knight, E. G. ed., Research monographs in cell and tissue physiology, Vol. 7, Liposomes: From physical structure to therapeutic applications, Elsevier, North-Holland, Biomedical Press, Amsterdam, New York, Oxford 1981). Wegen der Möglichkeit, sehr viele unterschiedliche Wirkstoffe in Vesikeln zu verkapseln, ist im Laufe der Zeit ein sehr breites Anwendungsspektrum entstanden, wobei ein Schwerpunkt bei der parenteralen Anwendung (i.v., s.c., i.m., i.p.) liegt. Weitere Einsatzgebiete von Vesikeln liegen in der Kosmetik, Nahrungsmittelindustrie oder Korrosionsbehandlung.

Die erfindungsgemäßen Vesikel-Dispersionen eignen sich ferner zum Einschluss von Wirkstoffen und zum Transport dieser Wirkstoffe durch die Haut und stellen daher ein wertvolles Trägersystem zur Anwendung kosmetischer und pharmakologischer Wirkstoffe dar, insbesondere eignen sie sich zum Einschluss wasserlöslicher Wirkstoffe in der von den Vesikeln eingeschlossenen wässrigen Phase. In den hydrophoben Teil der Vesikelmembran können aber auch lipidlösliche und wasserunlösliche Wirkstoffe eingebaut sein.

Wasserlösliche oder lipidlösliche kosmetische oder pharmazeutische und ernährungsphysiologische Wirkstoffe können in Vesikel aufgenommen werden, indem man die vesikelbildenden Moleküle mit den lipidlöslichen Wirkstoffen und gegebenenfalls mit einem wasserlöslichen Lösungsmittel mischt, die wasserlöslichen Wirkstoffe in Wasser löst und die Lipidphase in der wässrigen Phase dispergiert. Als wasserlösliche Wirkstoffe können z. B. wasserlösliche Vitamine, Mineralstoffe und Proteine, z. B. Salze der Ascorbinsäure, Aminosäuren und wasserlösliche Polypeptide, wasserlösliche Farbstoffe, antibakterielle Stoffe, entzündungshemmende Stoffe und viele andere eingesetzt werden. Als lipidlösliche Stoffe können z. B. öllösliche Vitamine, z. B. Tocopherole, UV-Filtersubstanzen oder andere wasserlösliche Wirksubstanzen eingeschlossen werden.

Als wasserlöslich werden dabei solche Wirkstoffe bezeichnet, die in Wasser bei 20 °C zu wenigstens 1 Gew.-% klar gelöst sind. Als öllöslich werden solche Stoffe verstanden, die in Paraffinöl bei 20 °C zu wenigstens 1 Gew.-% klar löslich sind. Die wässrige Phase kann die gelösten Stoffe dabei bis zur Sättigungsgrenze enthalten. Die Lipidphase, die im Wesentlichen aus den Membranlipiden besteht, sollte nicht mehr als 10 Gew.-% der Lipidphase, bevorzugt nicht mehr als 5 Gew.-% der Lipidphase an öllöslichen Wirkstoffen enthalten.

Die Erfindung wird nachstehend unter Bezugnahme auf die Ausführungsbeispiele näher erläutert, ohne sie darauf zu beschränken. In den Figuren zeigen
- Fig. 1a und 1b: Prinzipskizzen einer ersten Ausführungsform des erfindungsgemäßen Verfahrens, das in mit Hilfe einer Zentrifuge durchgeführt wird;
- Fig. 2a bis 2c: Prinzipskizzen einer zweiten Ausführungsform des erfindungsgemäßen Verfahrens, das mit Hilfe zweier Scherplatten durchgeführt wird;
- Fig. 3: eine Prinzipskizze einer dritten Ausführungsform des erfindungsgemäßen Verfahrens, das mittels Extrusion durchgeführt
- Fig. 4a bis 4d: Prinzipskizzen einer vierten Ausführungsform des erfindungsgemäßen Verfahrens, bei dem die Richtung der Strömungsgeschwindigkeit eines zweiten Fluids zeitlich variabel ist;
- Fig. 5a bis 5d: Prinzipskizzen einer fünften Ausführungsform des erfindungsgemäßen Verfahrens, bei dem die Strömungsgeschwindigkeit eines zweiten Fluids zeitlich konstant ist;
- Fig. 6: eine Prinzipskizze einer sechsten Ausführungsform des erfindungsgemäßen Verfahrens, bei dem ein zweites Fluid
- Fig. 7: eine Prinzipskizze einer siebten Ausführungsform des erfindungsgemäßen Verfahrens, bei dem die Richtung der Strömungsgeschwindigkeit eines ersten Fluids
- Fig. 8a und 8b: Prinzipskizzen einer achten Ausführungsform des erfindungsgemäßen Verfahrens, bei dem ein poröses Material einen sich verjüngenden Hohlraum begrenzt;
- Fig. 9: eine erste Ausführungsform der erfindungsgemäßen Vorrichtung mit einem oberen Block, einem unterm Block und einem sich dazwischen befindenden Spalt;
- Fig. 10: eine zweite Ausführungsform der erfindungsgemäßen Vorrichtung mit einem konischen Hohlkörper und einem konischen
- Fig. 11: eine dritte Ausführungsform der erfindungsgemäßen Vorrichtung mit einem Rohrresonator;
- Fig. 12: eine vierte Ausführungsform der erfindungsgemäßen Vorrichtung mit einem als Hohlzylinder ausgebildeten Hohlkörper und einer Welle.

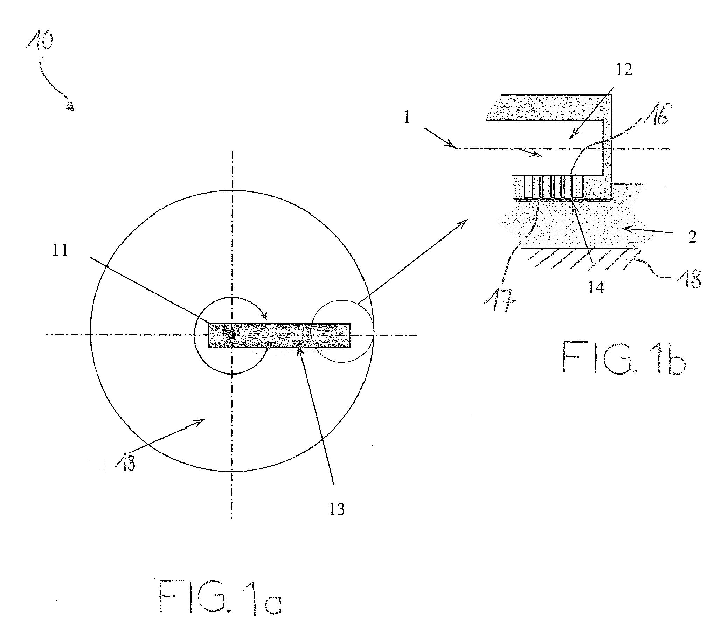

Bei der in den Figuren 1a und 1b gezeigten ersten Ausführungsform des erfindungsgemäßen Verfahrens wird eine Zentrifuge 10 verwendet. Gemäß der Draufsicht in Figur 1a enthält die Zentrifuge 10 eine horizontal ausgerichtete Platte 18 und einen rotierbaren Arm 13 mit einem ersten Kanal 12, welcher einen ersten Strömungsraum bildet. Ein die Ausgangsstoffe zur Vesikelbildung und einen oder mehrere Wirkstoffe enthaltendes erstes Fluid 1 wird über eine erste Einlassöffnung 11 in den ersten Kanal 12 zugeführt. Im Randbereich des Arms befindet sich ein poröses Material, das als Membran 14 ausgebildet ist. Die Membran 14 weist eine erste Seite 16 und eine zweite Seite 17 auf. Sie enthält Poren mit einem im Wesentlichen einheitlichen Durchmesser von 500 nm, welche sich von der ersten Seite 16 zur zweiten Seite 17 erstrecken.

Das erste Fluid 1 liegt in einer geordneten Phase vor, insbesondere in einer eindimensional lamellaren Phase. Diese geordnete Phase wurde durch geeignete Wahl von Temperatur und/oder pH-Wert und/oder Ionenzahl und/oder Ionenstärke des ersten Fluids (1) und/oder durch die Konzentrationen der Inhaltsstoffe im ersten Fluid (1) eingestellt. Die geeigneten Parameter können dabei vom Fachmann durch Routineversuche ermittelt werden. Das Vorliegen einer geordneten Phase, insbesondere einer eindimensional lamellaren Phase, kann etwa mithilfe Röntgenbeugung, Freeze-Fracture Electron Microscopy oder ³¹P-NMR bestimmt werden.
Durch eine nicht dargestellte, die Platte 18 durchdringende Eintrittsöffnung kann ein zweites Fluid von unten auf die Oberseite der Platte 18 befördert werden. Auf der Oberseite der Platte 18 bildet das zweite Fluid einen radial nach außen strömenden Film. Optional kann die Platte 18 um die vertikale Achse rotieren, wodurch die radiale Strömung unterstützt wird. Der Raumbereich oberhalb der Platte 18 bildet damit einen zweiten Strömungsraum. Der Arm 13 und insbesondere die Membran 14 tauchen in diesen Film ein.

Bei der Rotation des Arms 13 wird das erste Fluid 1 gemäß der Detailansicht in Figur 1b aufgrund von Zentrifugalkräften in den Randbereich des Arms 13 und durch die Poren von der ersten Seite 16 zur zweiten Seite 17 der Membran 14 gepresst, wo es in das zweite Fluid 2 gelangt. Das erste Fluid 1 bildet beim Austritt aus den Poren der Membran 14 und beim Eintritt in das zweite Fluid 2 Vesikel (nicht gezeigt) einer einheitlichen Größe und einer einheitlichen Beladung mit dem Wirkstoff.

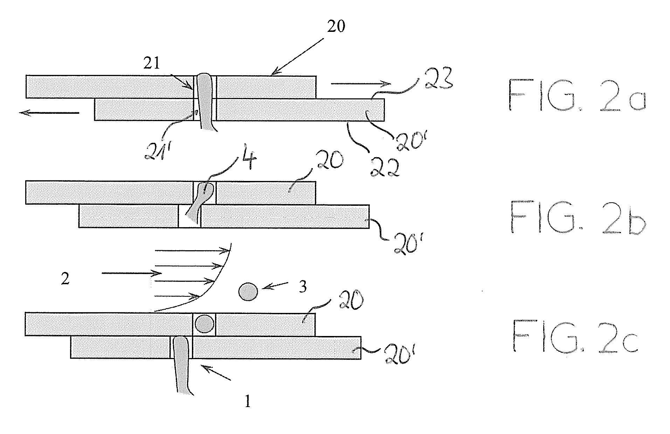

In den Figuren 2a bis 2c ist eine zweite Ausführungsform des erfindungsgemäßen Verfahrens dargestellt. Bei diesem Verfahren werden zwei Scherplatten 20, 20' verwendet. Jede der beiden Scherplatten 20, 20' enthält jeweils mindestens eine Pore 21 bzw. 21'. Die untere Scherplatte 20' bildet dabei ein poröses Material im Sinne der Erfindung und weist eine erste Seite 22 und eine zweite Seite 23 auf. Ein die Ausgangsstoffe zur Vesikelbildung und den einen oder mehrere Wirkstoffe enthaltendes erstes Fluid 1 wird gemäß Figur 2a durch die beiden Poren 21, 21' gepresst. Bei der in Figur 2b dargestellten Scherbewegung der beiden Scherplatten 20, 20' zueinander wird aus dem ersten Fluid 1 zunächst ein Tropfen 4 gebildet. Zu dem in Figur 2c dargestellten Zeitpunkt wurde der Tropfen entlang der zweiten Seite 23 der unteren Scherplatte 20' zur Bildung eines Vesikels 3 abgetrennt. Dieses Vesikel 3 wird von einem zweiten Fluid 2 abtransportiert. Durch dieses Verfahren werden Vesikel einer definierten Größe und definierten Beladung mit Wirkstoff erhalten.

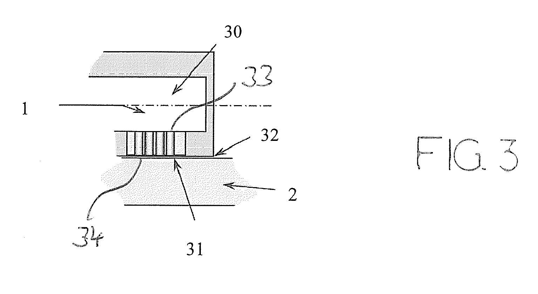

In Figur 3 ist eine dritte Ausführungsform des erfindungsgemäßen Verfahrens visualisiert, das mittels Extrusion durchgeführt wird. Ein die Ausgangsstoffe zur Vesikelbildung und den einen oder mehrere Wirkstoffe enthaltendes erstes Fluid 1 wird in einen Kanal 30 gepresst, welcher einen ersten Strömungsraum bildet. Am Ende des Kanals 30 befindet sich ein poröses Material, welches als Membran 31 ausgebildet ist. Die Membran 31 weist eine erste Seite 33 und eine zweite Seite 34 auf und enthält Poren mit einem im Wesentlichen einheitlichen Durchmesser von 500 nm. Das erste Fluid 1 wird mittels eines Druckgradienten durch die Poren von der ersten Seite 33 zur zweiter Seite 34 gepresst. An der zweiten Seite 34 der Membran 31 befindet sich ein zweiter Strömungsraum 32, in dem ein zweites Fluid 2 strömt. Beim Austritt aus den Poren der Membran 31 und beim Eintritt in das zweite Fluid 2 bildet das erste Fluid 1 Vesikel (nicht gezeigt) einer einheitlichen Größe und Beladung mit dem Wirkstoff. Der Druck p1 des ersten Fluids 1 ist hierbei größer als der Druck p2 des zweiten Fluids und derart auf beispielsweise die Porengrösse der Membran 31 und/oder die Viskositäten des ersten Fluids 1 und/oder des zweiten Fluids 2 abgestimmt, dass das erste Fluid 1 die Membran in einem gewünschten Durchsatz passieren kann.

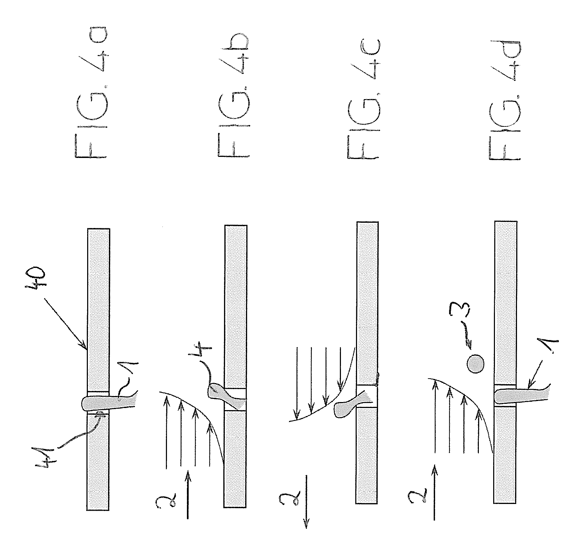

In den Figuren 4a bis 4d ist eine vierte Ausführungsform des erfindungsgemäßen Verfahrens wiedergegeben. Ein die Ausgangsstoffe zur Vesikelbildung und den einen oder mehrere Wirkstoffe enthaltendes erstes Fluid 1 wird gemäß Figur 4a durch eine oder mehrere in einem porösen Material 40 vorhandene Poren 41 aufwärts gepresst. Beim Durchführen des ersten Fluids 1 durch die Pore 41 wird zunächst ein Tropfen 4 gebildet. Durch pulsierenden Fluss eines zweiten Fluids 2 in verschiedenen Richtungen (vgl. Figuren 4b und 4c) wird in Figur 4d der Tropfen zur Bildung eines Vesikels 3 abgetrennt. Die somit entstehenden Vesikel 3 haben eine einheitliche Größe und Beladung mit Wirkstoff.

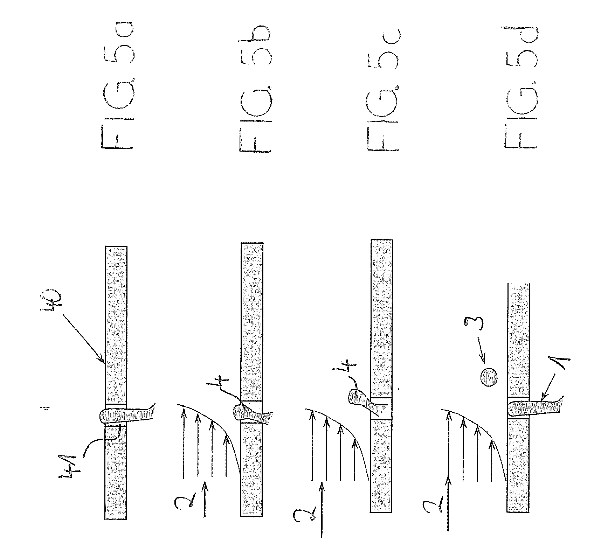

Die Figuren 5a bis 5d geben eine fünfte Ausführungsform des erfindungsgemäßen Verfahrens wieder. Dieses unterschiedet sich von dem in den Figuren 4a bis 4d dargestellten Verfahren lediglich dadurch, dass die Strömungsgeschwindigkeit des zweiten Fluids 2 zeitlich (aber nicht räumlich) konstant ist.

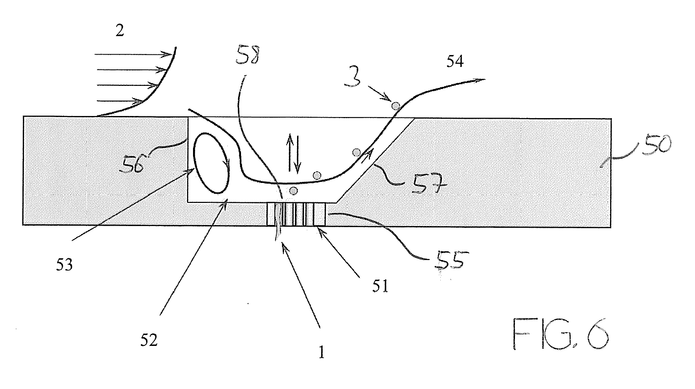

Figur 6 zeigt eine sechste Ausführungsform des erfindungsgemäßen Verfahrens. Ein die Ausgangsstoffe zur Vesikelbildung und den einen oder mehrere Wirkstoffe enthaltendes erstes Fluid 1 wird durch mehrere in einem porösen Material 55 befindliche Poren 51 (aufwärts) in eine Vertiefung 52 gepresst. Die Vertiefung 52 weist in Strömungsrichtung des zweiten Fluids 2 einen steilen Abfall 56 auf und steigt im weiteren Verlauf 57 flach an, so dass stromaufwärts von den Poren 51 eine Verwirbelung 53 gebildet wird. An der Grenzschicht 58 des mit Wellen und/oder Schwingungen beaufschlagten Flussfeldes 54 gebildete Vesikel 3 werden durch das zweite Fluid 2 abtransportiert.

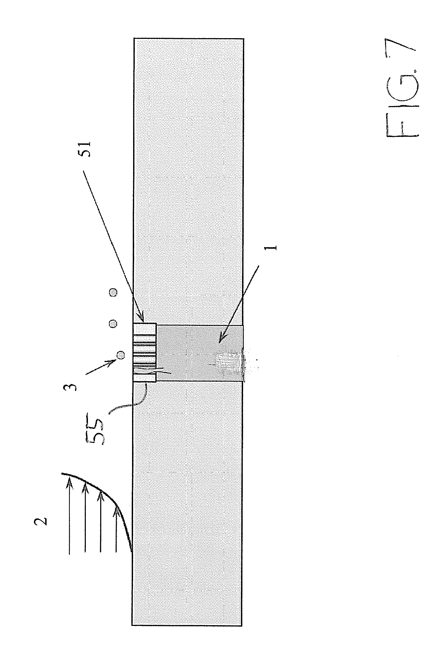

Eine siebte Ausführungsform des erfindungsgemäßen Verfahrens ist in Figur 7 erkennbar. Ein die Ausgangsstoffe zur Vesikelbildung und den einen oder mehrere Wirkstoffe enthaltendes erstes Fluid 1 mit zeitlich variabler (pulsierender) Strömungsgeschwindigkeit wird durch mehrere Poren 51 eines porösen Materials 55 gepresst, wodurch Vesikel 3 abgetrennt und in einem zweiten Fluid 2 abtransportiert werden. Die Strömungsgeschwindigkeit kann dabei zeitweise bis auf null absinken. Anstelle einer variablen Strömungsgeschwindigkeit des ersten Fluids 1 können auch Schwingungen des porösen Materials 55 verwirklicht werden, welche beispielsweise mittels eines Ultraschall-Resonators erzeugt werden können.

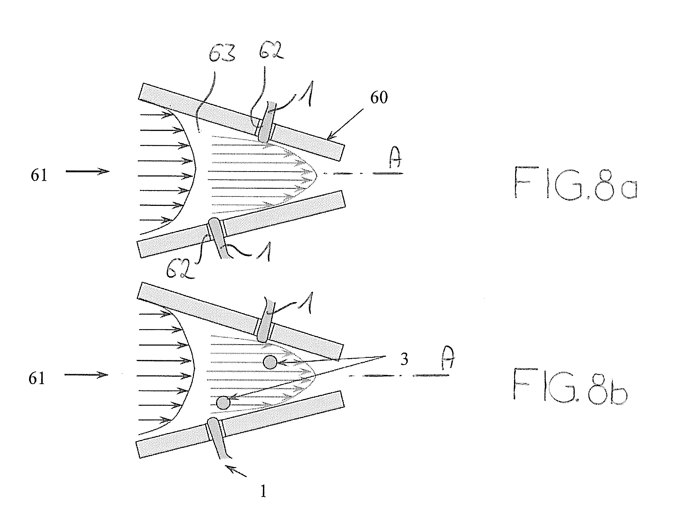

Weiterhin ist in den Figuren 8a und 8b eine achte Ausführungsform des erfindungsgemäßen Verfahrens dargestellt. Ein die Ausgangsstoffe zur Vesikelbildung und den einen oder mehrere Wirkstoffe enthaltendes erstes Fluid 1 wird durch mehrere sich in einer Fläche 60 befindliche Poren 62 gepresst. Die Fläche 60 begrenzt einen sich verjüngenden Hohlraum 63, welcher sich in einer Achsrichtung A verjüngt. Der Hohlraum 63 bildet einen zweiten Strömungsraum, den ein zweiten Fluid 2 in Achsrichtung durchströmt. Beim Pressen des ersten Fluids 1 durch die Poren 62 werden Vesikel 3 abgetrennt und in dem zweiten Fluid 2 abtransportiert, wie Figur 8b zeigt. Durch die Verjüngung des Hohlraums in Flussrichtung wird eine ursprüngliche Dehnungsströmung 61 erzeugt.

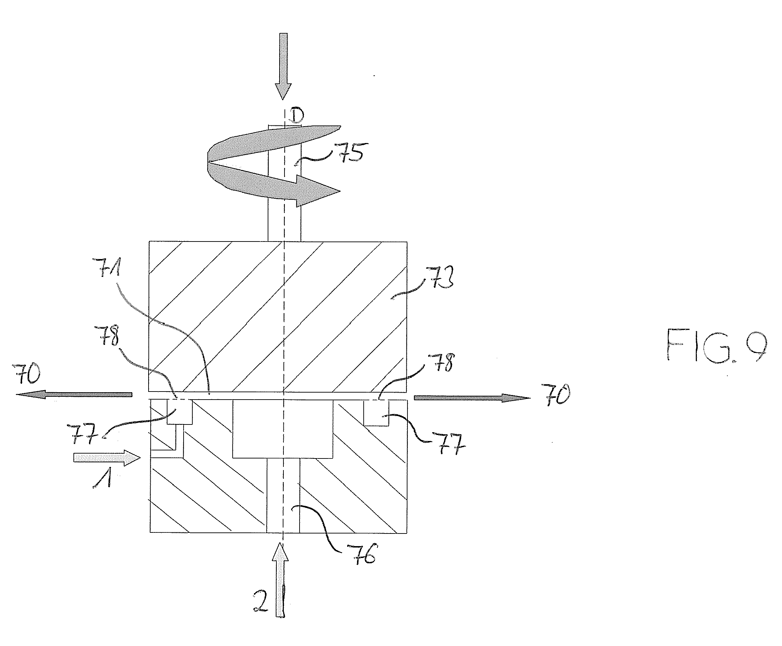

Die Figur 9 zeigt eine erste Ausführungsform einer erfindungsgemäßen Vorrichtung, die zur Durchführung des erfindungsgemäßen Verfahrens geeignet ist. Die Vorrichtung umfasst einen oberen Block 73 und einen unteren Block 74, zwischen denen ein Spalt 71 gebildet ist. Mittels einer am oberen Block angeordneten Welle 75 kann die Breite des Spaltes 71 eingestellt werden. Außerdem kann der obere Block 73 mittels der Welle 75 gegenüber dem unteren Block 74 um eine vertikale Drehachse D in Rotation versetzt werden.

Im unteren Block 74 ist eine erste Einlassöffnung in Form eines Ringkanals 77 bereitgestellt, über die ein erstes Fluid 1 an einer ersten Stelle in den Spalt 71 gelangen kann. Weiterhin ist im unteren Block 74 eine zentral in axialer Richtung vorliegende zweite Einlassöffnung 76 für ein zweites Fluid 2 vorgesehen, über die das zweite Fluid 2 an einer zweiten Stelle in den Spalt 71 gelangen kann. Diese zweite Stelle ist von der ersten Stelle verschieden und radial weiter innen angeordnet ist als die erste Stelle. Der Spalt 71 bildet einen zweiten Strömungsraum für das zweite Fluid 2. Die Strömung im Spalt 71 entspricht der Strömung in einem Axiallager. Der Spalt 71 sowie die seitlichen Begrenzungen des oberen Blocks 73 und des unteren Blocks 74 bilden eine zweite Auslassöffnung 70 für das zweite Fluid 2.

An den Einlassöffnungen des ersten Fluids 1 in den Spalt 71 sind Membranen 78 vorgesehen, welche ein poröses Material bilden.

Die Breite des Spaltes 71 bildet eine einstellbare Dimension des zweiten Strömungsraumes, und die den Spalt 71 nach oben begrenzende untere Oberfläche des ersten Blocks 73 bildet eine bewegbare, den zweiten Strömungsraum begrenzende Fläche. Zur Einstellung der Breite des Spaltes 71 muss die Membran 78 nicht bewegt werden, was die Zuführung des ersten Fluids 1 sehr vereinfacht. Die Breite des Spaltes 71 kann durch die axiale Kraft an der Welle 75 oder durch den Volumenstrom des zweiten Fluids 2 eingestellt werden. Die Scherrate resultiert aus der Drehzahl und der Einstellung der Breite des Spaltes 71.

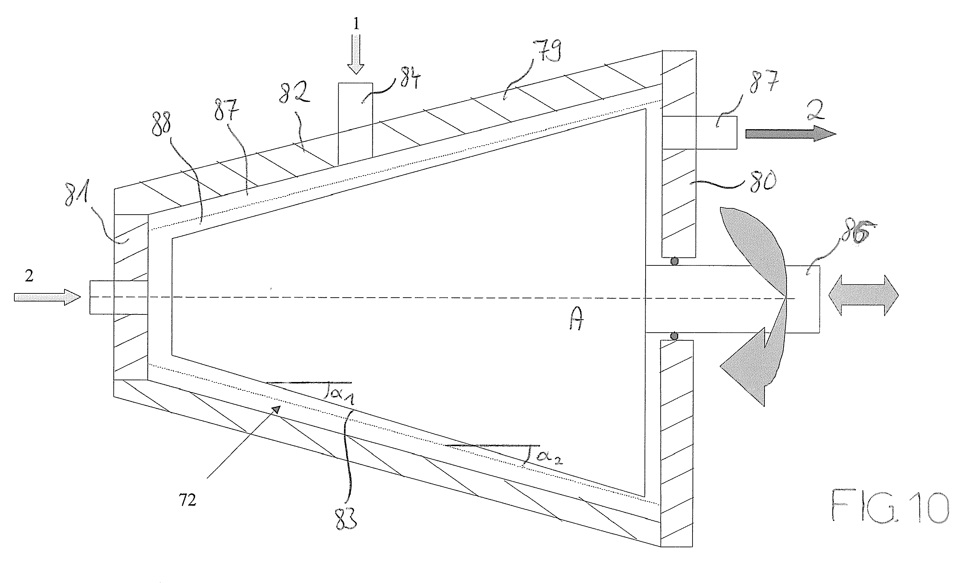

In Figur 10 ist eine Seitenansicht einer weiteren Ausführungsform einer erfindungsgemäßen Vorrichtung dargestellt, die zur Durchführung eines erfindungsgemäßen Verfahrens geeignet ist. Ein konischer Hohlkörper 79 mit Grundfläche 80, Deckfläche 81 und Mantelfläche 82 umfasst eine zur inneren Mantelfläche 82 des Holkörpers 79 gleichmäßig beabstandete Membran 72 und einen darin angeordneten konischen Innenkörper 83. Der konische Innenkörper 83 weist einen Öffnungswinkel α₁ auf; die Membran 72 weist einen Öffnungswinkel α₂ auf, welcher kleiner ist als α₁. Hierdurch verengt sich der Spalt zwischen dem konischen Innenkörper 83 und der Membran 72 in Richtung der Grundfläche 80. In der Mantelfläche 82 ist eine erste Einlassöffnung 84 für ein erstes Fluid 1 angeordnet, während in der Deckfläche 81 eine zweite Einlassöffnung 85 für das zweite Fluid 2 angeordnet ist. In der Grundfläche 80 ist ferner eine zweite Auslassöffnung 87 für das zweite Fluid 2 angeordnet. Der erste Strömungsraum 87 ist zwischen der Membran 72 und dem konischen Hohlkörper 79 angeordnet, und der zweite Strömungsraum 88 ist zwischen dem konischen Innenkörper 83 und der Membran 72 angeordnet.

Die Grundfläche 80 umfasst eine Welle 86 zum Verschieben des konischen Innenkörpers 83 entlang der horizontalen Symmetrieachse A, um den Abstand zwischen dem konischen Innenkörper 83 und der Membran 72 zu ändern. Der Abstand zwischen dem konischen Innenkörper 83 und der Membran 72 bildet im Sinne der Erfindung eine einstellbare Dimension des zweiten Strömungsraumes, während der konische Oberfläche des konischen Innenkörpers 83 die bewegbare, den zweiten Strömungsraum begrenzende Fläche darstellt. Zur Einstellung der Breite des Spaltes zwischen dem konischen Innenkörper 83 und der Membran 72 muss die Membran 72 nicht bewegt werden, was die Zuführung des ersten Fluids 1 sehr vereinfacht.
Ferner ist der konische Innenkörper 83 mittels der Welle 86 auch um die Symmetrieachse A herum drehbar, beispielsweise mittels eines nicht dargestellten Motors. Durch konstruktive Ausbildung eines kleinen Öffnungswinkels α₁ und/oder α₂ kann die axiale Kraft auf die Konusse minimiert werden. Die Scherrate resultiert aus dem Abstand zwischen dem konischen Innenkörper 83 und der Membran sowie der Drehzahl der Welle 86.

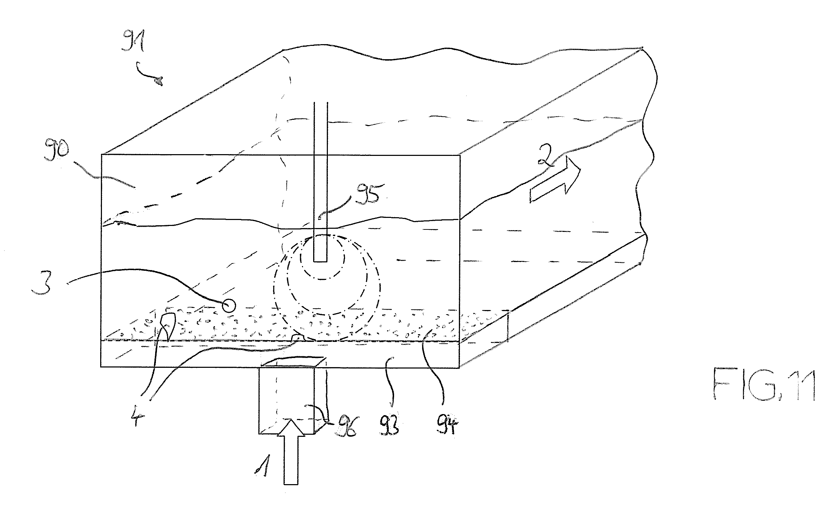

Die Figur 11 zeigt eine dritte Ausführungsform der erfindungsgemäßen Vorrichtung. Die Vorrichtung enthält einen Kanal 91, welcher einen ersten Strömungsraum 93 für ein erstes Fluid 1 und einen zweiten Strömungsraum 90 für ein zweites Fluid 2 umfasst. Zwischen dem ersten Strömungsraum 93 und dem zweiten Strömungsraum 90 ist ein poröses Material angeordnet, welches als Membran 94 ausgebildet ist. An einer Wandfläche 92 des Kanals 91 ist eine erste Einlassöffnung 96 angeordnet, durch welche das erste Fluid 1 in den ersten Strömungsraum 93 eintreten kann. Das erste Fluid 1 wird durch die Poren der Membran 94 vom ersten Strömungsraum 93 zum zweiten Strömungsraum 90 geführt. Hierbei bilden sich zunächst Tropfen 4 aus dem ersten Fluid 1. Mittels des zweiten Fluids 2 werden diese Tropfen 4 zur Bildung von Vesikeln 3 abgetrennt. Das Abtrennen der Tropfen 4 wird von einem Rohrresonator 95 unterstützt, welcher im zweiten Strömungsraum 90 angeordnet ist. Der Rohrresonator 95 wird dabei mit einer Frequenz zwischen 2 Hz und 100 Hz, vorzugsweise zwischen 10 Hz und 20 Hz angeregt.

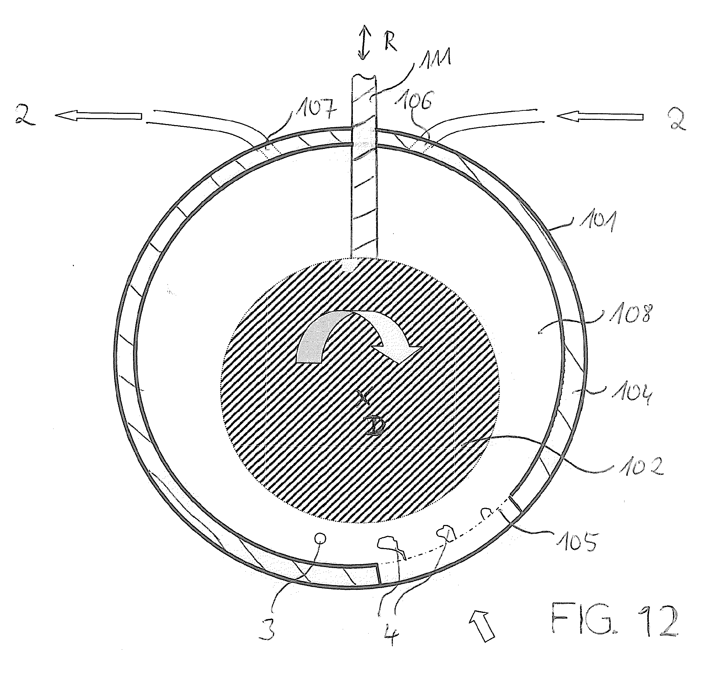

Schließlich zeigt Figur 12 eine seitliche Schnittansicht einer vierten Ausführungsform einer erfindungsgemäßen Vorrichtung zum Abscheren von Tropfen. Diese Vorrichtung umfasst einen als Hohlzylinder 101 ausgebildeten Hohlkörper und eine innerhalb des Hohlzylinders 101 angeordnete Welle 102, welche um eine horizontale Drehachse D drehbar ist. Der Hohlzylinder 101 weist eine Mantelfläche 104 mit einer Membran 105 auf, welche vorzugsweise im Bereich des engsten Spaltes zwischen der Welle 102 und dem Hohlzylinder 101 angeordnet ist. Die Welle 102 ist an einer Führungsplatte 111 gehalten, welche die Mantelfläche 104 des Hohlzylinders 101 durchdringt. Die Führungsplatte 111 ist in beiden durch den Doppelpfeil gezeigten Richtungen R bewegbar, also senkrecht zur Mantelfläche 104. Zwischen der Membran 105 und der Welle 102 ist ein Spalt 110 gebildet. Durch Bewegung der Welle 102 mittels Bewegung der Führungsplatte 111 ist die Breite dieses Spaltes 110 einstellbar. Zur Einstellung der Breite des Spaltes 110 muss die Membran 105 nicht bewegt werden, was die Zuführung des ersten Fluids 1 sehr vereinfacht.

Durch die Membran 105 wird das erste Fluid 1 eingebracht. Das zweite Fluid 2 wird an einer der Strömungsrichtung vorgelagerten zweiten Einlassöffnung 106 an der Mantelfläche 104 eingebracht. Die im zweiten 2 Fluid suspendierten Vesikel 3 werden dann durch den Spalt 110 geführt und an einer der Strömungsrichtung nachgelagerten zweiten Auslassöffnung 107 aus dem Hohlraum 108 zwischen Mantelfläche 104 und Welle 102 herausgeführt. Dieser Hohlraum 108 bildet also einen zweiten Strömungsraum für das zweite Fluid 2. Die Führungsplatte 111 verhindert ein direktes Strömen des zweiten Fluids von der zweiten Einlassöffnung 106 zur zweiten Auslassöffnung 107.

Die Welle 102 ist mittels Bewegung der Führungsplatte 111 exzentrisch gegenüber dem Hohlzylinder 101 verschiebbar und kann mit der gewünschten Umfangsgeschwindigkeit gedreht werden. Durch entsprechende Wahl der Durchmesser der Welle 102 und des Hohlzylinders 101 und durch Variation der Drehzahl und der Exzentrizität der Welle 102 lassen sich die gewünschten Dehn-und Scherströmungen im zweiten Fluid 2 einstellen, welche das Abtrennen der Tropfen 4 zur Bildung von Vesikeln 3 hervorrufen.

## Patentansprüche

1. Verfahren zur Herstellung von Vesikeln (3), umfassend:
(a) Bereitstellen eines ersten Fluids (1), welches zumindest eine amphiphile Substanz enthält;
(b) Durchführen des ersten Fluids (1) durch mindestens eine Pore (21;21';41;51;62) von einer ersten Seite (16;22;33) eines porösen Materials (14;31;40;55;72;78;94;105), insbesondere einer Membran (14;31;72;78;94;105), zu einer zweiten Seite (17;34) des porösen Materials (14;31;40;55;72;78;94;105);
(c) Bildung mindestens eines Tropfens (4) aus dem ersten Fluid (1) an der zweiten Seite (17;34) des porösen Materials (14;31;40;55;72;78;94;105);
(d) Bildung mindestens eines Vesikels (3) durch Abtrennung, insbesondere Abscherung des Tropfens (4) mittels Vorbeiführens eines zweiten Fluids (2) an der zweiten Seite (17;23;34) des porösen Materials (14;31;40;55;72;78;94;105), wobei die chemische Zusammensetzung des zweiten Fluids (2) von der chemischen Zusammensetzung des ersten Fluids (1) verschieden ist.

2. Verfahren nach Anspruch 1,
**dadurch gekennzeichnet, dass**
das erste Fluid (1) mindestens einen Inhaltsstoff in einer ersten Konzentration enthält und das zweite Fluid (2) den Inhaltsstoff in einer zweiten Konzentration enthält, die von der ersten Konzentration verschieden ist, insbesondere kleiner ist als die erste Konzentration, wobei insbesondere der Inhaltsstoff ein Wirkstoff ist.

3. Verfahren nach einem der vorstehenden Ansprüche,
**dadurch gekennzeichnet, dass**
während Schritt (d) das erste Fluid (1) und/oder das zweite Fluid (2) und/oder das poröse Material (14;31;40;55;72;78;94;105) in mechanische Schwingungen versetzt und/oder mit mechanischen Wellen beaufschlagt werden.

4. Verfahren nach einem der vorstehenden Ansprüche,
**dadurch gekennzeichnet, dass**
die amphiphile Substanz mindestens ein Block-Copolymer umfasst, insbesondere mindestens ein Block-Copolymer mit mindestens einer Ester-Funktionalität und/oder mindestens einer Ether-Funktionalität.

5. Verfahren nach Anspruch 4,
**dadurch gekennzeichnet, dass**
mindestens ein amphiphiles Block-Copolymer Poly(alkylenoxid)ₙ-Poly(alkylcaprolacton)ₘ ist, insbesondere Poly(ethylenoxid)ₙ-Poly(alkylcaprolacton)ₘ und/oder Poly(alkylenoxid)ₙ-Poly(methylcaprolacton)ₘ und/oder Poly(ethylenoxid)ₙ-Poly(methylcaprolacton)ₘ.

6. Verfahren gemäß Anspruch 5, wobei n im Bereich von 10 bis 50 liegt und/oder m im Bereich von 20 bis 80 liegt.

7. Verfahren gemäß einem der Ansprüche 5 und 6, wobei mindestens ein amphiphiles Block-Copolymer eine Struktur nach folgender Formel I aufweist:

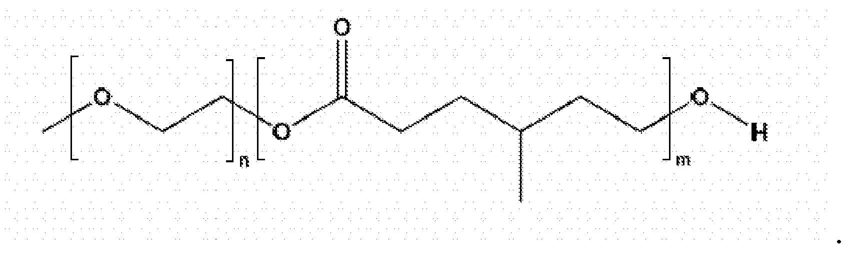

8. Verfahren gemäß einem der Ansprüche 4 bis 7,
**dadurch gekennzeichnet, dass**
mindestens ein amphiphiles Block-Copolymer Poly(isobutylen)ₙ-Poly(ethyloxid)ₘ ist, wobei insbesondere n im Bereich von 30 bis 100 liegt und insbesondere m im Bereich von 10 bis 50 liegt, und insbesondere eine Struktur nach folgender Formel II aufweist:

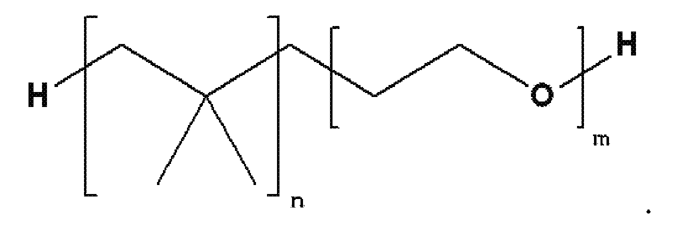

9. Gesamtzusammensetzung enthaltend ein Trägerfluid und mindestens ein Vesikel (3), insbesondere mindestens ein mit einem Verfahren gemäß einem der vorangehenden Ansprüche hergestelltes Vesikel (3), wobei die chemische Zusammensetzung des Vesikels (3) von der chemischen Zusammensetzung des Trägerfluids verschieden ist, insbesondere das Vesikel (3) mindestens einen Inhaltsstoff in einer ersten Konzentration enthält und das Trägerfluid den Inhaltsstoff in einer zweiten Konzentration enthält, die von der ersten Konzentration verschieden ist, insbesondere kleiner ist als die erste Konzentration, bevorzugt weniger als 80 %, weiter bevorzugt weniger als 50 %, besonders bevorzugt weniger als 30 % der ersten Konzentration beträgt, und wobei insbesondere die pH-Werte im Innenbereich des Vesikels (3) und im Trägerfluid im Wesentlichen gleich sind.

10. Vorrichtung zur Herstellung von Vesikeln (3), insbesondere in einem Verfahren gemäß einem der Ansprüche 1 bis 8, umfassend
- mindestens ein poröses Material
(14;31;40;55;72;78;94;105), insbesondere eine Membran (14;31;72;78;94;105), und/oder eine Aufnahme für ein poröses Material (14;31;40;55;72;78;94;105), insbesondere für eine Membran (14;31;72;78;94;105), wobei das poröse Material (14;31;40;55;72;78;94;105) eine erste Seite (16;22;33) und eine zweite Seite (17;23;34) sowie mindestens eine Pore (21;21';41;51;62) aufweist, welche sich von der ersten Seite (16;22;33) zur zweiten Seite (17;23;34) erstreckt;
- einen ersten Strömungsraum (12;30;93) zum Heranführen eines ersten Fluids (1) an die erste Seite (16;22;33) des porösen Materials (14;31;40;55;72;78;94;105);
- einen zweiten Strömungsraum (15;32;63;71;90;108) zum Vorbeiführen eines zweiten Fluids (2) an der zweiten Seite (17;23;34) des porösen Materials (14;31;40;55;72;78;94;105),
wobei mindestens eine Dimension des zweiten Strömungsraumes (15;32;63;71;90;108) einstellbar ist zumindest mittels einer Bewegung mindestens einer den zweiten Strömungsraum (15;32;63;71;90;108) begrenzenden Fläche relativ zur zweiten Seite (17;23;34), insbesondere mindestens eine Dimension des zweiten Strömungsraumes (15;32;63;71;90;108) im Bereich der zweiten Seite (17;23;34) und im Wesentlichen senkrecht zur zweiten Seite (17;23;34), insbesondere die Breite eines Spaltes (71;110) des zweiten Strömungsraumes (15;32;63;71;90;108) im Bereich der zweiten Seite (17;23;34).

11. Vorrichtung gemäß Anspruch 10, umfassend
- einen Hohlkörper, insbesondere einen Hohlzylinder (101) mit
o einer Mantelfläche (104), welche den zweiten Strömungsraum (108) umschließt und an welcher das poröse Material (105) angeordnet ist;
o einer zweiten Einlassöffnung (106) für das zweite Fluid (2), welche mit dem zweiten Strömungsraum (108) in Verbindung steht;
o und einer zweiten Auslassöffnung (107) für das zweite Fluid (2), welche mit dem zweiten Strömungsraum (108) in Verbindung steht;
- eine innerhalb des Hohlkörpers angeordnete drehbare Welle (102),
wobei
- die Welle (102) mit insbesondere variabler Umfangsgeschwindigkeit relativ zum Hohlkörper drehbar ist, wodurch die Welle (102) die Mittel zur Herstellung einer Scherströmung, einer Dehnströmung oder einer beliebiger Kombination davon im zweiten Fluid (2) bildet,
- die Welle (102) gegenüber dem Holkörper, insbesondere gegenüber einer Längsachse eines Hohlzylinders (101), exzentrische verschiebbar.

12. Vorrichtung zur Herstellung von Vesikeln (3), insbesondere in einem Verfahren gemäß einem der Ansprüche 1 bis 8, insbesondere Vorrichtung gemäß einem der Ansprüche 10 und 11, oder Vorrichtung zur Herstellung einer Gesamtzusammensetzung gemäß Anspruch 9, umfassend
- mindestens ein poröses Material
(14;31;40;55;72;78;94;105), insbesondere eine Membran (14;31;72;78;94;105), und/oder eine Aufnahme für ein poröses Material (14;31;40;55;72;78;94;105), insbesondere für eine Membran (14;31;72;78;94;105), wobei das poröse Material (14;31;40;55;72;78;94;105) eine erste Seite (16;22;33) und eine zweite Seite (17;23;34) sowie mindestens eine Pore (21;21';41;51;62) aufweist, welche sich von der ersten Seite (16;22;33) zur zweiten Seite (17;23;34) erstreckt;
- einen ersten Strömungsraum (12;30;93) zum Heranführen eines ersten Fluids (1) an die erste Seite (16;22;33) des porösen Materials (14;31;40;55;72;78;94;105);
- einen zweiten Strömungsraum (15;32;63;71;90;108) zum Vorbeiführen eines zweiten Fluids (2) an der zweiten Seite (17;23;34) des porösen Materials (14;31;40;55;72;78;94;105),
**gekennzeichnet durch**
Mittel zum Beaufschlagen des ersten Fluids (1) und/oder des zweiten Fluids (2) und/oder des porösen Materials 14;31;40;55;72;78;94;105) mit mechanischen Schwingungen und/oder Wellen, insbesondere enthaltend einen Rohrresonator (95) und/oder einen Piezofilm und/oder einen Unwuchterzeuger und/oder einen Elektromagnet.

13. Verwendung einer Vorrichtung zur Herstellung von Vesikeln (3) und/oder zur Herstellung einer Gesamtzusammensetzung gemäß Anspruch 9, insbesondere in einem Verfahren gemäß einem der Ansprüche 1 bis 8, insbesondere Vorrichtung gemäß einem der Ansprüche 10 bis 12, wobei die Vorrichtung aufweist:
- mindestens ein poröses Material
(14;31;40;55;72;78;94;105), insbesondere eine Membran (14;31;72;78;94;105), und/oder eine Aufnahme für ein poröses Material (14;31;40;55;72;78;94;105), insbesondere für eine Membran (14;31;72;78;94;105), wobei das poröse Material (14;31;40;55;72;78;94;105) eine erste Seite (16;22;33) und eine zweite Seite (17;23;34) sowie mindestens eine Pore (21;21';41;51;62) aufweist, welche sich von der ersten Seite (16;22;33) zur zweiten Seite (17;23;34) erstreckt;
- einen ersten Strömungsraum (12;30;93) zum Heranführen eines ersten Fluids (1) an die erste Seite (16;22;33) des porösen Materials (14;31;40;55;72;78;94;105);
- einen zweiten Strömungsraum (15;32;63;71;90;108) zum Vorbeiführen eines zweiten Fluids (2) an der zweiten Seite (17;23;34) des porösen Materials
(14; 31; 40; 55; 72; 78; 94; 105).

14. Verwendung von Vesikeln (3), welche mit einem Verfahren nach einem der Ansprüche 1 bis 8 und/oder mit einer Vorrichtung gemäß einem der Ansprüche 10 bis 12 erzeugt wurden, oder Verwendung einer Gesamtzusammensetzung gemäß Anspruch 9 als oder in einem Medikament, einem Kosmetikum, einem Nahrungsmittel, einer Farbe, einem Kraftstoff oder einem Antikorrosionsmittel.
